# EUROPEAN PATENT SPECIFICATION

(11) **EP 3 846 938 B1**
(45) Date of publication and mention of the grant of the patent: **07.08.2024**
(21) Application number: 19858319.7
(22) Date of filing: 03.09.2019
(51) Int. Cl.: B01J 37/025, C12N 9/04, C12N 11/14

(54) **IMMOBILIZED ENZYMES AND MICROSOMES ON MAGNETIC SCAFFOLDS**
IMMOBILISIERTE ENZYME UND MIKROSOMEN AUF MAGNETISCHEN GERÜSTEN
ENZYMES IMMOBILISÉES ET MICROSOMES SUR DES ÉCHAFAUDAGES MAGNÉTIQUES

(30) Priority: 05.09.2018 US 201862727519 P
(43) Date of publication of application: 14.07.2021
(73) Proprietor: ZYMtronix Catalytic Systems, Inc., Ithaca, NY 14853 (US)
(72) Inventor: CORGIE, Stephane Cedric, Ithaca, New York 14853 (US); CHUN, Matthew Stephen, Ithaca, New York 14853 (US); HOEPKER, Alexander Chris, Ithaca, New York 14853 (US); RIVERA, Katia Argelia Rodriguez, Ithaca, New York 14853 (US); WONG, Braedon Carter, Ithaca, New York 14853 (US)
(74) Representative: Kilburn & Strode LLP
(86) International application number: PCT/US2019/049397
(87) International publication number: WO 2020/051159

(56) References cited:
- WO-A1-2014/055853
- WO-A1-2016/138477
- WO-A1-2018/102319
- WO-A2-2004/065616
- S C CORGIé ET AL: "Self-Assemblies of Magnetic Nanoparticles (MNPs) and Peroxidase Enzymes: Mesoporous Structures and Nanoscale Magnetic Field Effects (nano-MFEs) for Enhanced Activity BioNanoCatalysts (BNCs)", 31 December 2012 (2012-12-31), XP055554952, Retrieved from the Internet <URL:https://briefs.techconnect.org/wp-content/volumes/Cleantech2012/pdf/179.pdf> [retrieved on 20190211]
- ZHENG, M ET AL.: "Magnetic field intensified bi-enzyme system with in situ cofactor regeneration supported by magnetic nanoparticles", JOURNAL OF BIOTECHNOLOGY, vol. 168, no. 2, 10 June 2013 (2013-06-10), pages 212 - 217, XP028742469

## Description

### FIELD OF THE INVENTION

The present invention provides devices and methods for producing metabolites used in measuring the toxicity of chemical compounds. They incorporate enzymatic microsomes and magnetic nanoparticles that magnetically entrap enzymes. These enzyme systems catalyze chemicals to yield measurable metabolic products. The microsomes and the magnetic nanoparticles containing enzymes are associated with macroporous scaffolds and non-reactive components that facilitate the enzyme reactions.

### BACKGROUND OF THE INVENTION

Many drugs and chemicals that are developed may themselves be safe but may be metabolized to toxins. Thus, a critical aspect of drug and chemicals development is to ensure that unsafe compounds "fail fast" by establishing the compound's safety well before it ever enters the human testing phase or consumer markets. With about 2,000 new chemicals being commercialized each year, a key part of quickly determining a chemical's safety is to assess the contribution of potentially toxic metabolites that the body produces from otherwise safe parent chemicals. Capturing such chemical metabolites (or breakdown products) and screening them for toxicity is therefore vital to ensure the safety of commercial chemicals, including pharmaceuticals. Currently, there are limited tools to quickly assess *in vitro* metabolism. It has therefore been a longstanding goal to retrofit existing tox-screening assay platforms with multiple human xenobiotic metabolic enzymes.

Magnetic enzyme immobilization involves the entrapment of enzymes in mesoporous magnetic clusters that self-assemble around the enzymes. The immobilization efficiency depends on a number of factors that include the initial concentrations of enzymes and nanoparticles, the nature of the enzyme surface, the electrostatic potential of the enzymes, the nanoparticle surface, and the time of contact. Enzymes used for industrial or medical manufacturing in biocatalytic processes should be highly efficient and stable before and during the process, reusable over several biocatalytic cycles, and economical. Enzymes used for screening and testing drugs or chemicals should be stable, reliable, sensitive, economical, and compatible with high-throughput automation.

P450-catalyzed reactions may be used to determine the toxicity of test compounds. Cytochrome P450 (referred to as P450 or CYP) are of the E.C. 1.14 class of enzymes. (Br. J. Pharmacol. 158(Suppl 1): S215-S217 (2009).) They constitute a family of monooxygenases involved in the biotransformation of drugs, xenobiotics, alkanes, terpenes, and aromatic compounds. They also participate in the metabolism of chemical carcinogens and the biosynthesis of physiologically relevant compounds such as steroids, fatty acids, eicosanoids, fat-soluble vitamins, and bile acids. Furthermore, they are also involved in the degradation of xenobiotics in the environment such pesticides and other industrial organic contaminants. They function by incorporating one hydroxyl group into substrates found in many metabolic pathways. In this reaction, dioxygen is reduced to one hydroxyl group and one H₂O molecule by the concomitant oxidation of a cofactor such as NAD(P)H.

Monooxygenases are key enzymes that act as detoxifying biocatalysts in all living systems and initiate the degradation of endogenous or exogenous toxic molecules. Phase I metabolism of xenobiotics includes functionalization reactions such as oxidation, reduction, hydrolysis, hydration and dehalogenation. Cytochrome P450 monooxygenases represent the most important class of enzymes involved in 75-80% of metabolism. Other phase I enzymes include monoamine oxidases, Flavin-containing oxygenases, amidases and esterases.

Phase II metabolism involves conjugation reactions (glucuronidation, sulfation, GSH conjugation, acetylation, amino acid conjugation and methylation) of polar groups (*e.g.* glucuronic acid, sulfate, and amino acids) on phase I metabolites.

In recent years there has been an increasing interest in the application of P450 biocatalysts. P450s, and most metabolic oxidative enzymes in general, require a cofactor for the conversion of their target compounds. Protons (H⁺) are usually delivered from the cofactor NADH or NADPH through specific amino acids in the CYP enzyme. They relay the protons to the active site where they reductively split an oxygen molecule so that a single atom can be added to the substrate. CYP enzymes receive electrons from a range of different redox partner enzymes including, but not limited to, glucose dehydrogenase (GDH) and formate dehydrogenase (FDH).

GDH (E.C. 1.1.1.47) catalyzes the oxidation of β-D-glucose to β-D-1,5-lactone with simultaneous reduction of NADP+ to NADPH or of NAD+ to NADH. FDH (EC 1.2.1.2) refers to a set of enzymes that catalyze the oxidation of formate to carbon dioxide. They donate electrons to a second substrate such as NAD+. These enzymes, especially from eukaryotic sources, have total-turnover numbers amongst the lowest of any enzymes. Biocatalytic reactions with cytochromes P450 are highly inefficient because substrate oxidation is associated with the production of Reactive Oxygen Species (ROS), *e.g.,* hydrogen peroxide and superoxide, as by-products. For eukaryotic monooxygenases, a large fraction of the activated oxygen from the enzymes are diverted from the oxidation of the targets and converted to ROS by either decay of the one-electron-reduced ternary complex that produces a superoxide anion radical (O-2), while the protonation of the peroxycytochrome P450 and the four-electron reduction of oxygen produce H₂O₂. Hence, eukaryotic P450 enzymes lose a very substantial part (>30%) of the consumed reducing equivalents for the production of ROS.

Compared to eukaryotic P450, bacterial P450s are more efficient as less than 10% of the total electron intake is diverted to ROS resulting in better efficiency of O₂ and electron conversion efficiency in the oxidation route. Special designs in bioreactors are necessary to control dissolved oxygen concentrations at levels that prevent the buildup of ROS without slowing down the reactions.

Oxidative inhibition due to the production of reactive oxidative species (ROS) is one of the major limitations of P450 biocatalysis. Reactive Oxygen Species (ROS) are a major by-product of the metabolic reactions of P450s and other oxidases including NADPH Oxidase (NOX), Lipoxygenase (LOX) and cyclooxygenase (COX). Reactive oxygen species (ROS) include highly reactive oxygen radicals [superoxide (02 •-), hydroxyl (•OH), peroxyl (RO2 •), alkoxyl (RO•)] and non-radicals that are either oxidizing agents and/or are easily converted into radicals. Examples include hypochlorous acid (HOCl), ozone (O₃), singlet oxygen (1O₂), and hydrogen peroxide (H₂O₂) as hydrogen peroxide (H₂O₂) and superoxide ion (O₂₋) if the reaction occurs in an excess of oxygen. High levels of ROS not only reduce the efficiency of the conversion reactions but also inhibit the reactions due to oxidative denaturation. One way to prevent ROS build up during an oxidative reaction is to scavenge key intermediaries using ROS degrading enzymes such as catalases or superoxide dismutases (SOD). They decontaminate the ROS while producing dioxygen and recycle oxygen radicals that can be used for the P450 oxidation cycles.

Other metabolic enzymes known in the art that produce metabolites in Phase I, II and III metabolism include UDP-glucuronosyl transferases, sulfotransferases, flavin-containing monooxygenases, monoamine oxidases, and carboxyesterases. Metabolic enzymes have low activity and are particularly unstable *ex-vivo.* In order to get high and fast production of chemical metabolites for screening or in biochemical production, the concentration of P450s has historically been high (50 to 200% substrate loading). In order to increase the oxidation rate of the target compounds, oxygen levels also need to be high at over-stoichiometric concentrations. This leads to the production of superoxide anions that denature the enzymes and limit the efficiency of the reaction.

Most metabolic processing systems used in life sciences and pharmaceutical markets are based on cultured *in vitro* hepatocytes (*e.g.,* HepG, HepaRG). The advantage of cell-based platforms is the ability to co-activate multiple biochemical networks consisting of systems of metabolic enzymes (CYPs, UDP-glucoronosylltransferases (UGTs), carboxyesterases, etc.) in a single integrated platform. Metabolic processing with such high biological complexity increases the chance of mimicking the liver physiology. Unfortunately, *in vitro* cell-based systems are vulnerable to cytotoxic effects imparted by metabolite buildup and cell stress by prolonged oxidative processing. This results in short incubation times (1-2 hours) and significantly lower enzymatic rates (~100-fold) than corresponding cell-free enzymatic systems. In addition, cellular systems produce a significant background metabolome that complicates the identification of substrate-derived metabolites by LC-MS analysis.

The art needs efficient methods for measuring compound toxicity for screening thousands of chemicals enzymatically. Metabolic enzymes need to be configured in devices and methods to facilitate high-throughput toxicity screens.

### SUMMARY OF THE INVENTION

The present invention is as set out in the appended claims. The technical information set out below may in some respects go beyond the scope of the present invention, which is defined by the appended claims. The additional technical information is provided to place the present invention in a broader technical context and to illustrate possible related technical developments.

The present invention provides devices and methods for producing metabolites used in measuring the toxicity of chemical compounds. They incorporate enzymatic microsomes and magnetic nanoparticles that magnetically entrap enzymes. These enzyme systems catalyze chemicals to yield measurable metabolic products. The microsomes and the magnetic nanoparticles containing enzymes are associated with macroporous scaffolds and non-reactive components that facilitate the enzyme reactions.

Thus, the present invention provides a device in accordance with the claims, comprising microsomes and self-assembled mesoporous aggregates of magnetic nanoparticles, wherein a first enzyme requiring a diffusible cofactor having a first enzymatic activity is contained within the microsomes, wherein a second enzyme comprising a cofactor regeneration activity is magnetically-entrapped within the mesopores, wherein the cofactor is utilized in the first enzymatic activity; wherein the first and second enzymes function by converting a diffusible substrate into a diffusible product; wherein the magnetic nanoparticles are magnetically associated with a macroporous scaffold; wherein the microsomes are associated with the macroporous scaffold; and wherein the macroporous scaffold comprising the magnetic nanoparticles is associated with a non-reactive portion operable for placing the macroporous scaffold into or removing it from a reaction solution.

In some embodiments of the invention, the device further comprises a functional portion for stably maintaining the microsomes, the magnetic nanoparticles, the first enzyme, the second enzyme, and the macroporous scaffold. In preferred embodiments, the functional portion comprises a buffer. In other preferred embodiments, the functional portion comprises a substrate for the second enzyme. In other preferred embodiments, the functional portion comprises the cofactor. In other preferred embodiments, the functional portion is magnetic.

In some embodiments of the invention, the co-factor is entrapped in the mesoporous aggregates of magnetic nanoparticles with the first and second enzymes.

In some embodiments of the invention, the macroporous scaffold is in the shape of a cylindrical pin, an orb, a bead, a capsule, a cube, a squared rod, a pyramid, a diamond, or is amorphous. In a preferred embodiment, the macroporous scaffold is in the shape of a cylindrical pin.

In some embodiments of the invention, the non-reactive portion comprises metal, plastic, ceramic, a composite, or a combination thereof. In other embodiments, the non-reactive portion comprises a handle for manipulating the device. In other embodiments, the non-reactive portion is operable for handling by a robotic arm for high-throughput screening. In other embodiments, the non-reactive portion allows for diffusion of gases.

In some embodiments of the invention, the mesoporous aggregates of magnetic nanoparticles have an iron oxide composition. In other embodiments, the mesoporous aggregates of magnetic nanoparticles have a magnetic nanoparticle size distribution in which at least 90% of the magnetic nanoparticles have a size of at least 3 nm and up to 30 nm, and an aggregated particle size distribution in which at least 90% of the mesoporous aggregates of magnetic nanoparticles have a size of at least 10 nm and up to 500 nm.

In some embodiments of the invention, the mesoporous aggregates of magnetic nanoparticles possess a saturated magnetization of at least 10 emu/g. In other embodiments, the mesoporous aggregates of magnetic nanoparticles possess a remnant magnetization up to 5 emu/g.

In some embodiments of the invention, the first and second enzymes are contained in the mesoporous aggregates of magnetic nanoparticles in up to 100% of saturation capacity.

In some embodiments of the invention, the first and second enzymes are physically inaccessible to microbes.

In some embodiments of the invention, the first enzyme is an oxidative enzyme. In preferred embodiments, the oxidative enzyme is a Flavin-containing oxygenase; wherein the composition further comprises a third enzyme having a co-factor reductase activity that is co-located with the first enzyme. In other preferred embodiments, the oxidative enzyme is a P450 monooxygenase; wherein the composition further comprises a third enzyme having a co-factor reductase activity that is co-located with the first enzyme.

In some embodiments of the invention, a single protein comprises the P450 monooxygenase and the third enzyme. In other embodiments, the P450 monooxygenase is co-located with the third enzyme within a lipid membrane.

In some embodiments of the invention, the third enzyme is a cytochrome P450 reductase. In other embodiments, the P450 monooxygenase comprises a P450 sequence that is mammalian.

In a preferred embodiment, the P450 monooxygenase comprises a P450 sequence that is human. In another preferred embodiment, the P450 monooxygenase comprises CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1,CYP3A4, CYP3A5, CYP3A7, CYP3A43,CYP4A11, CYP4A22, CYP4B1, CYP4F2, CYP4F3, CYP4F8, CYP4F11, CYP4F12, CYP4F22, CYP4V2, CYP4X1, CYP4Z1,CYP5A1,CYP7A1, CYP7B1,CYP8A1, CYP8B1,CYP11A1, CYP11B1, CYP11B2, CYP17A1, CYP19A1, CYP20A1, CYP21A2, CYP24A1, CYP26A1, CYP26B1, CYP26C1, CYP27A1, CYP27B1, CYP27C1, CYP39A1, CYP46A1, or CYP51A1.

In some embodiments of the invention, the P450 monooxygenase comprises a P450 sequence that is of an origin selected from the group consisting of primate, mouse, rat, dog, cat, horse, cow, sheep, and goat. In other embodiments, the P450 monooxygenase comprises a P450 sequence that is of an origin selected from the group consisting of insect, fish, fungus, yeast, protozoan, and plant.

In some embodiments of the invention, the second enzyme is selected from the group consisting of a carbonyl reductase, an aldehyde dehydrogenase, an aryl-alcohol dehydrogenase, an alcohol dehydrogenase, a pyruvate dehydrogenase, a D-1 xylose dehydrogenase, an oxoglutarate dehydrogenase, an isopropanol dehydrogenase, a glucose-6-phosphate dehydrogenase, a glucose dehydrogenase, a malate dehydrogenase, a formate dehydrogenase, a benzaldehyde dehydrogenase, a glutamate dehydrogenase, and an isocitrate dehydrogenase.

In some embodiments of the invention, the cofactor is nicotinamide adenine dinucleotide + hydrogen (NADH), nicotinamide adenine dinucleotide phosphate + hydrogen (NADPH), Flavin adenine dinucleotide + hydrogen (FADH), or glutathione.

In some embodiments of the invention, the first enzyme participates in phase I metabolism.

Some embodiments of the invention provide a third enzyme that participates in phase II metabolism.

Some embodiments of the invention provide a fourth enzyme that reduces a reactive oxygen species (ROS). In other embodiments, the fourth enzyme is a catalase, a superoxide dismutase (SOD), or a glutathione peroxidase/glutathione-disulfide reductase or a combination thereof.

Some embodiments of the invention provide a fifth enzyme selected from the group consisting of a UDP-glucoronosyl transferase, a sulfotransferase, a monoamine oxidase, and a carboxyl esterase.

In some embodiments of the invention, the macroporous scaffold is a magnetic macroporous scaffold. In other embodiments, the macroporous magnetic scaffold is a polymeric hybrid scaffold comprising a cross-linked water-insoluble polymer and an approximately uniform distribution of embedded magnetic microparticles (MMP). In other embodiments, the magnetic macroporous polymeric hybrid scaffold comprises PVA and a polymer selected from the group consisting of CMC, alginate, HEC, EHEC. In other embodiments, the magnetic macroporous polymeric hybrid scaffold comprises a hydrophilic polymer. In other embodiments, the hydrophilic polymer is xanthan gum.

In some embodiments of the invention, one or more the enzymes are produced by recombinant DNA technology. In other embodiments, one or more the enzymes are synthesized.

In some embodiments of the invention, the magnetic nanoparticles comprise human liver microsomes (HLM) or enzymes from a human liver cytosol fraction (HLCF).

The present invention provides a method of producing metabolites of a compound, comprising mixing said compound with a diffusible substrate in a reaction solution, positioning the device in accordance with the claims such that said macroporous scaffold comprising the magnetic nanoparticles contacts said reaction solution, and measuring a product resulting from an enzymatic reaction in said reaction solution.

In some embodiments, the method further comprises the step of removing the macroporous scaffold comprising the microsomes and the magnetic nanoparticles from the solution. In other embodiments, the method is incorporated into a high-throughput screening method for screening the toxicity of a plurality of compounds. In other embodiments, the method is incorporated into a high-throughput screening method for screening metabolites from mixtures of metabolic enzymes.

The invention provides a method of manufacturing the device disclosed herein, comprising magnetically entrapping the second enzyme in the mesoporous aggregates of magnetic nanoparticles, combining the aggregates with the second enzyme with the microsomes comprising the first enzyme, templating the aggregates and microsomes onto the macroporous scaffolds, and templating the scaffolds onto the non-reactive portion.

In some embodiments of the methods disclosed herein, the aggregates further comprise a third enzyme having a co-factor reductase activity. In other embodiments, the aggregates further comprise a fourth enzyme that is a catalase, a superoxide dismutase (SOD), or a glutathione peroxidase/glutathione-disulfide reductase. In other embodiments, the aggregates further comprise a fifth enzyme that participates in phase II metabolism.

### BRIEF DESCRIPTION OF THE DRAWINGS

**Figure 1A** Shows a pin for use with a 2 ml tube before functionalization with a macroporous functional tip. **Figure 1B** shows a drawing of a pin functionalized with a macroporous scaffold.
**Figure 2A** is a diagram of the side view of a microplate 96-pin array. **Figure 2B** is a diagram of a bottom perspective of a microplate 96-pin array.
**Figure 3A** and **Figure 3B** show pictures of functionalized pins from different perspectives. **Figure 3C** shows a functionalized pin in a 2 ml tube.
**Figure 4A** shows a scanning electron micrograph (SEM) of a side view of the coating material with a diamond shape. **Figure 4B** shows an SEM of the side surface. **Figure 4C** shows an SEM of the top surface. **Figure 4D** shows an SEM of the poor wall.

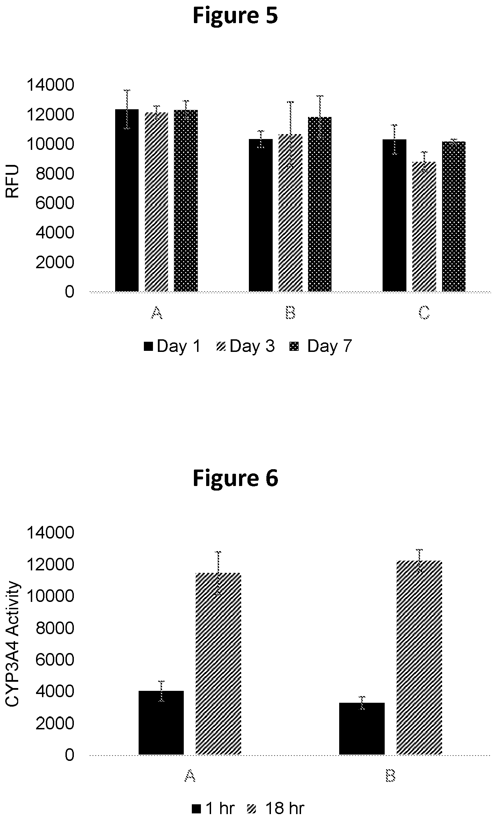

**Figure 5** storage and stability analyses of immobilized CYP3A4 using a fluorometric assay that measures dealkylation of 7-ethoxyresorufin (7-ER) to resorufin (RFN). The first set of bars (A) represent immobilized CYP3A4 stored at 4° C. The second set of bars (B) show storage at -20° C. The third set of bars (C) show storage at -80° C.
**Figure 6** shows CYP3A4 activity after 1 hour and 18 hour incubation times at 37° C using a self-sufficient enzymatic system that includes glucose-6-phosphate dehydrogenase (G6DH), Catalase (CAT), superoxide dismutase (SOD), and immobilized NADP. Immobilization conditions: (1) 250 µg/ml nanoparticles at pH 11, and (2) 1000 µg/ml nanoparticles at pH 11. Stability was shown over 7 days.

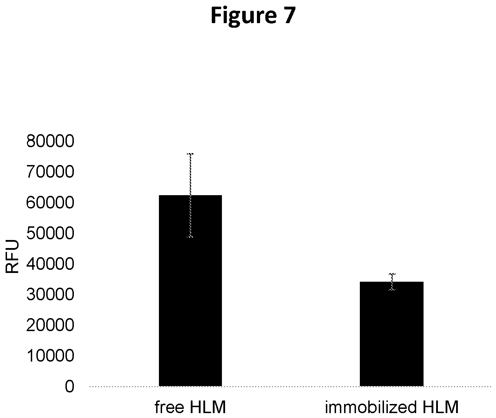

**Figure 7** shows CYP3A4 activity of free and immobilized human liver microsome in a 1 hour reaction at 37°C.
**Figure 8A** shows the immobilization yield of CYP2B6 on <10µm magnetite powder and the relative activity of the immobilized CYP2B6 compared to the free CYP2B6 at the same protein concentration. **Figure 8B** shows the immobilized CYP2B6 and the free CYP2B6 activity measured in luminescence units.
**Figure 9** Immobilized CYP2B6 retained greater than or equal to 50% of the activity of fresh free CYP2B6.
**Figure 10A** shows the immobilization yield of UGT1A6 on <10µm magnetite powder and the relative activity of the immobilized UGT1A6 compared to the free UGT1A6 at the same protein concentration. **Figure 10B** shows the immobilized UGT1A6 and the free UGT1A6 activity measured in luminescence units.
**Figure 11** shows immobilized UGT1A6 retained greater than or equal to 50% of the activity of fresh free UGT1A6.
**Figure 12A** shows the immobilization yield of CYP3A4 as determined in duplicate to be 97%±0.4%, 91%±5%, and 92%±0.5% for immobilization buffer pH 7.0, pH 7.5, and pH 8.0 respectively.
**Figure 12B** shows the immobilized CYP3A4 activity relative to the free CYP3A4 enzyme as determined to be 35%±3%, 30%±3%, and 36%±5% for immobilization buffer pH7.0, pH 7.5, and pH 8.0 respectively at the same concentration of protein.
**Figure 13A** shows immobilized CYP3A4 stored at -20°C retained 46%±4%, 39%±3%, 46%±1%, and 53%±3% activity relative to free CYP3A4 over 1, 3, 7, and 14 days respectively. Immobilized CYP3A4 stored at -20°C retained 63%±6%, 53%±4%, 62%±1%, and 72%±4% relative to freshly immobilized CYP3A4 over 1, 3, 7, and 14 days respectively.
**Figure 13B** shows immobilized CYP3A4 freeze dried and then stored at 4°C retained 18%±12%, 23%±1%, 23%±0%, and 19%±4% activity relative to free CYP3A4 over 1, 3, 7, and 14 days respectively. Immobilized CYP3A4 freeze dried and then stored at 4°C retained 25%±16%, 31%±2%, 31%±0.3%, and 25%±6% relative to freshly immobilized CYP3A4 over 1, 3, 7, and 14 days respectively (Figure 13B).

### DETAILED DESCRIPTION OF THE INVENTION

The present invention provides devices and methods for producing metabolites to subsequently measure the toxicity of chemical compounds. The microsomes and the magnetic nanoparticle-containing enzymes are associated with macroporous scaffolds and non-reactive components that facilitate the enzyme reactions.

The invention provides a universal platform for immobilizing and stabilizing enzymes for biocatalytic processes. The cell-free technology works by the molecular entrapment of enzymes or enzymatic mixtures within self-assembling nanoparticle (NP) clusters templated onto magnetic carriers without the need for protein modifications or covalent coupling. Ionic strength, buffer pH, and NP concentration are the main parameters in controlling the cluster size and immobilization yields. The magnetic materials allow for self-assembly at different scales (nano to macro) for precise control of enzyme loading, stability, and activity. Corgie et al., Catalysis & Biocatalysis - Chemistry Today 34(5):15-20 (2016). This technology is now expanded to cell-free metabolic profiling for downstream high-throughput toxicological screening with immobilized CYPs and self-sufficient metabolic enzyme systems.

Cell-free metabolic profiling has several advantages over a cell-based approach. Because cell-free enzymes are not limited by competing background cell maintenance and proliferation processes, enzymatic conversion rates in a cell-free system (e.g., Corning Supersomes https://www.corning.com/worldwide/en/products/life-sciences/products/adme-tox-research/recombinant-metabolic-enzymes.html) typically yield 10-fold higher amounts of metabolites than in human primary hepatocytes (hPH) - the "gold standard" for metabolism, clearance, and hepatotoxicity. This is measured over a 1-2-hour incubation time. In addition, the cytotoxic load of substrates, metabolites and reactive oxygen species (ROS) restrict metabolic longevity.

Another advantage is Metabolic polymorphism. The ability to add and maintain controlled ratios of enzymatic components enables the formulation of tissue/organ specific metabolic profiles and addresses the genetic diversity of human metabolism. In addition, low, medium, and high metabolic activity levels can be readily accessed by adjusting enzyme concentrations. In addition to liver metabolism, metabolic enzyme constructs of other organs or body compartments can also be readily accessed. This includes the gastrointestinal tract, or even microbiomes, by adding bacterial metabolic enzymes.

Another advantage is assay design and handling. Cell-free enzyme reaction systems have a simple and controlled composition (buffer, salt, sugar, cofactor) compared to complex cell growth media (*e.g.,* DMEM). Cleaner chemical profiles simplify data and pattern analysis for more robust statistics. The absence of cellular processes produces a low background metabolome that is specific to the parent substrate and the selected enzyme(s), resulting in more robust dose-response profiles and especially at longer incubation times.

In some embodiments, the devices and methods disclosed herein are used in cell-free toxicological screens. In other embodiments, they are used in parallel with cell-based solutions. In some embodiments, cell-based assays are used as a broad first screen for toxic metabolites. Cell-free metabolite screening may then be used as a refined mechanistic approach to identify metabolites out of the base-case range. Combined with HT screening, cell-free profiling with metabolic enzyme combinations can produce an array of metabolomes from each chemical substrate that (1) quickly captures the polymorphism of human metabolomes and (2) allows for the elucidation of metabolic products of complex metabolic pathways. The invention supports the reduction, refinement, or replacement of animals in toxicity testing and the early-stage awareness of potential toxic metabolites in out-of-normality metabolic profiles.

Commercially available cell-free metabolic enzymes, including human liver microsomes (HLM), human-recombinant microsomes, and purified human-recombinant CYP monooxygenases, are notoriously unstable and retain their activity for incubation periods of only 1-2 hours. Human liver microsomes are also rare and expensive as they are produced from pooled liver microsomes fractions originating from cadavers that represent the population. Human CYPs in particular have a very low total-turnover number due to the production of inhibitory reactive oxygen species (ROS)-hydrogen peroxide and superoxide-as by-products. Extending the metabolic activity of CYPs is a principal barrier to producing enough metabolite quantities to ensure adequate analytical detection, especially in the case of low clearance metabolites.

In some embodiments, the invention produces chemical metabolites with stabilized synthetic enzyme systems immobilized on macroporous scaffolds designed to be compatible with robotic high-throughput (HT) toxicity assays or chemical analytics. In other embodiments, the macroporous scaffolds are not designed for robotic HT assays, but rather, smaller-scale benchtop analyses.

Self-assembled mesoporous nanoclusters comprising magnetically-immobilized enzymes are highly active and stable prior to and during use. Magnetically immobilized enzymes do not require bonding agents for incorporation into the self-assembled mesopores formed by the magnetic nanoparticles (MNPs or NPs). No permanent chemical modifications or crosslinking of the enzymes to the MNPs are required. The technology is a blend of biochemistry, nanotechnology, and bioengineering at three integrated levels of organization: Level 1 is the self-assembly of enzymes, and in some cases, cofactors, with MNPs for the synthesis of magnetic mesoporous nanoclusters. This level uses a mechanism of molecular self-entrapment to immobilize enzymes and cofactors. An enzyme immobilized in self-assembled magnetic nanoparticles is herein referred to as a "bionanocatalyst" (BNC). Level 2 is the stabilization of the MNPs or microsomes into other assemblies such as magnetic or polymeric matrices. In certain embodiments, the BNCs or microsomes are "templated" onto or into micro or macro structures for commercial or other applications. In one embodiment, the level 2 template is a Magnetic Microparticle (MMP). Level 3 is product conditioning for using the Level 1+2 immobilized enzymes.

In one embodiment, the first step is combining CYP microsomes and a level 1 G6DH-ROS recycling enzyme system. Glucose 6-phosophate dehydrogenase (G6DH) is co-immobilized to ensure co-localization in the cluster for an efficient NADPH recycling system. G6DH ensures availability of NADPH over longer incubation times (*e.g.* 18 hours). Nanomolar concentrations of catalase (CAT) and superoxide dismutase (SOD) are co-immobilized to ensure reactive oxygen species (ROS) scavenging. This combination ensures the co-localization of all the enzymes required for self-sufficient reactions.

In this embodiment, the second step stabilizes level 1 and microsomes on level 2 magnetic materials. The NP clusters (level 1) are templated via magnetic self-assembly on larger porous magnetic scaffolds (level 2) to prevent over-aggregation, to stabilize the clusters and enzymes, and to allow ease-of-handling of the immobilized enzymes. Level 2 magnetic materials need to be suitable for microsome adsorption by maintaining the integrity of the phospholipid membranes. Microsomes are efficiently adsorbed to level 2 by electrostatic and/or hydrophobic interactions.

In some embodiments, recombinant human CYP enzyme systems are immobilized on 3D printed magnetic pins for metabolic processing in small volumes. The pins have a non-reactive portion for support or handling. In other embodiments, the pins are in 96-pin arrays for metabolic profiling in 96-well microplates compatible with robotic handling and downstream assays. Additive manufacturing, also known as 3D printing, is an emerging industrial method of production that lends itself to at-scale biotechnological applications by enabling designs and compositions not accessible with other manufacturing methods. The immobilization stabilizes the enzymes, makes them easy to use ("plug, incubate, analyze"), and prevents them from entering the product stream.

In other embodiments, the cell-free metabolism of subject chemicals is done with combinations of pin-immobilized microsomes. In preferred examples, the microsome combinations may include CYP3A4, CYP2B6, CYP2E1, UGT1A6, in combination with level 1 CAT, SOD and GDH. CYP3A4 is a member of the Cytochrome P450 family of enzymes. Enzymes in this family are monooxygenases involved in drug metabolism.

In some embodiments, the invention provides printable 3D pins for microplate applications. In preferred embodiments, a level 2 scaffold powder material of high macroporosity composed of magnetite (particle size 100 nm, 50% w/w) is embedded in freeze-dried polyvinyl alcohol-cellulose crosslinked composites (referred to as monolith scaffolds). In some embodiments, the powders required an external automated magnetic mixer. In other embodiments, CYPs are immobilized in cylindrical pins (*e.g.* 3.6 mm Ø, 10 mm height) printed by stereolithography (SLA) from methacrylate resin (Formlabs https://formlabs.com/store/us/form-2/materials/clear-resin/). In preferred embodiments, they are designed for 0.5 ml metabolic reaction volumes in 2.0 ml microtubes. In other embodiments, a permanent magnet (1/16" Ø, 1/8" height) at the tip of the pin allows the capture of the level 2 monolith carrier (up to 45 mg) containing the metabolic enzyme systems.

In some embodiments, the BNCs used in the invention are provided in a magnetic macroporous polymeric hybrid scaffold comprising a cross-linked water-insoluble polymer and an approximately uniform distribution of embedded magnetic microparticles (MMP). The polymer comprises at least polyvinyl alcohol (PVA), has MMPs of about 50-500nm in size, pores of about 1 to about 50 µm in size, about 20% to 95% w/w MMP, wherein the scaffold comprises an effective surface area for incorporating bionanocatalysts (BNC) that is about total 1-15 m²/g; wherein the total effective surface area for incorporating the enzymes is about 50 to 200 m²/g; wherein the scaffold has a bulk density of between about 0.01 and about 10 g/ml; and wherein the scaffold has a mass magnetic susceptibility of about 1.0×10⁻³ to about 1×10⁻⁴ m³/kg. In a preferred embodiment, the magnetic macroporous polymeric hybrid scaffold comprises a contact angle for the scaffold with water that is about 0-90 degrees.

In preferred embodiments, the cross-linked water-insoluble polymer is essentially polyvinyl alcohol (PVA). In more preferred embodiments, the scaffold further comprises a polymer selected from the group consisting of polyethylene, polypropylene, poly-styrene, polyacrylic acid, polyacrylate salt, polymethacrylic acid, polymethacrylate salt, polymethyl methacrylate, polyvinyl acetate, polyvinylfluoride, polyvinylidenefluoride, polytetrafluoroethylene, a phenolic resin, a resorcinol formaldehyde resin, a polyamide, a polyurethane, a polyester, a polyimide, a polybenzimidazole, cellulose, hemicellulose, carboxymethyl cellulose (CMC), 2-hydroxyethylcellulose (HEC), ethylhydroxyethyl cellulose (EHEC), xylan, chitosan, inulin, dextran, agarose, alginic acid, sodium alginate, polylactic acid, polyglycolic acid. a polysiloxane, a polydimethylsiloxane, and a polyphosphazene.

In other more preferred embodiments, the magnetic macroporous polymeric hybrid scaffold comprises PVA and CMC, PVA and alginate, PVA and HEC, or PVA and EHEC. Macroporous polymeric hybrid scaffolds are taught in U.S. Prov. App. No. 62/323,663.

The MNPs allow for a broader range of operating conditions for using enzymes in biocatalytic processes such as temperature, ionic strength, pH, and solvents. The size and magnetization of the MNPs affect the formation and structure of the BNCs. This has a significant impact on the activity of the entrapped enzymes. By virtue of their surprising resilience under various reaction conditions, self-assembled MNP clusters can be used as a superior immobilization material for enzymes that replaces polymeric resins, cross-linked gels, cross-linked enzyme aggregates (CLEAs), cross-linked magnetic beads and the like. Furthermore, they can be used in any application of enzymes on diffusible substrates.

BNC's contain mesopores that are interstitial spaces between the clustered magnetic nanoparticles. Enzymes are immobilized within at least a portion of the mesopores of the magnetic BNCs. As used herein, the term "magnetic" encompasses all types of useful magnetic characteristics, including permanent magnetic, superparamagnetic, paramagnetic, and ferromagnetic behaviors.

BNC sizes used in the invention are in the nanoscale, *i.e.,* generally no more than 500 nm. As used herein, the term "size" can refer to a diameter of the magnetic nanoparticle when the magnetic nanoparticle is approximately or substantially spherical. In a case where the magnetic nanoparticle is not approximately or substantially spherical (e.g., substantially ovoid or irregular), the term "size" can refer to either the longest dimension or an average of the three dimensions of the magnetic nanoparticle. The term "size" may also refer to the calculated average size in a population of magnetic nanoparticles.

In different embodiments, the magnetic nanoparticle has a size of precisely, about, up to, or less than, for example, 500 nm, 400 nm, 300 nm, 200 nm, 100 nm, 50 nm, 40 nm, 30 nm, 25 nm, 20 nm, 15 nm, 10 nm, 5 nm, 4 nm, 3 nm, 2 nm, or 1 nm, or a size within a range bounded by any two of the foregoing exemplary sizes.

Within BNCs, the individual magnetic nanoparticles may be primary nanoparticles (*i.e.,* primary crystallites) having any of the sizes provided above. The aggregates of nanoparticles in a BNC are larger in size than the nanoparticles and generally have a size (*i.e.,* secondary size) of at least about 5 nm. In different embodiments, the aggregates have a size of precisely, about, at least, above, up to, or less than, for example, 5 nm, 8 nm, 10 nm, 12 nm, 15 nm, 20 nm, 25 nm, 30 nm, 35 nm, 40 nm, 45 nm, 50 nm, 60 nm, 70 nm, 80 nm, 90 nm, 100 nm, 150 nm, 200 nm, 300 nm, 400 nm, 500 nm, 600 nm, 700 nm, or 800 nm, or a size within a range bounded by any two of the foregoing exemplary sizes.

Typically, the primary and/or aggregated magnetic nanoparticles or BNCs thereof have a distribution of sizes, i.e., they are generally dispersed in size, either narrowly or broadly dispersed. In different embodiments, any range of primary or aggregate sizes can constitute a major or minor proportion of the total range of primary or aggregate sizes. For example, in some embodiments, a particular range of primary particle sizes (for example, at least about 1, 2, 3, 5, or 10 nm and up to about 15, 20, 25, 30, 35, 40, 45, or 50 nm) or a particular range of aggregate particle sizes (for example, at least about 5, 10, 15, or 20 nm and up to about 50, 100, 150, 200, 250, or 300 nm) constitutes at least or above about 50%, 60%, 70%, 80%, 90%, 95%, 98%, 99%, or 100% of the total range of primary particle sizes. In other embodiments, a particular range of primary particle sizes (for example, less than about 1, 2, 3, 5, or 10 nm, or above about 15, 20, 25, 30, 35, 40, 45, or 50 nm) or a particular range of aggregate particle sizes (for example, less than about 20, 10, or 5 nm, or above about 25, 50, 100, 150, 200, 250, or 300 nm) constitutes no more than or less than about 50%, 40%, 30%, 20%, 10%, 5%, 2%, 1%, 0.5%, or 0.1% of the total range of primary particle sizes.

The aggregates of magnetic nanoparticles (i.e., "aggregates") or BNCs thereof can have any degree of porosity, including a substantial lack of porosity depending upon the quantity of individual primary crystallites they are made of. In particular embodiments, the aggregates are mesoporous by containing interstitial mesopores (i.e., mesopores located between primary magnetic nanoparticles, formed by packing arrangements). The mesopores are generally at least 2 nm and up to 50 nm in size. In different embodiments, the mesopores can have a pore size of precisely or about, for example, 2, 3, 4, 5, 10, 12, 15, 20, 25, 30, 35, 40, 45, or 50 nm, or a pore size within a range bounded by any two of the foregoing exemplary pore sizes. Similar to the case of particle sizes, the mesopores typically have a distribution of sizes, i.e., they are generally dispersed in size, either narrowly or broadly dispersed. In different embodiments, any range of mesopore sizes can constitute a major or minor proportion of the total range of mesopore sizes or of the total pore volume. For example, in some embodiments, a particular range of mesopore sizes (for example, at least about 2, 3, or 5, and up to 8, 10, 15, 20, 25, or 30 nm) constitutes at least or above about 50%, 60%, 70%, 80%, 90%, 95%, 98%, 99%, or 100% of the total range of mesopore sizes or of the total pore volume. In other embodiments, a particular range of mesopore sizes (for example, less than about 2, 3, 4, or 5 nm, or above about 10, 15, 20, 25, 30, 35, 40, 45, or 50 nm) constitutes no more than or less than about 50%, 40%, 30%, 20%, 10%, 5%, 2%, 1%, 0.5%, or 0.1% of the total range of mesopore sizes or of the total pore volume.

The magnetic nanoparticles can have any of the compositions known in the art. In some embodiments, the magnetic nanoparticles are or include a zerovalent metallic portion that is magnetic. Some examples of such zerovalent metals include cobalt, nickel, and iron, and their mixtures and alloys. In other embodiments, the magnetic nanoparticles are or include an oxide of a magnetic metal, such as an oxide of cobalt, nickel, or iron, or a mixture thereof. In some embodiments, the magnetic nanoparticles possess distinct core and surface portions. For example, the magnetic nanoparticles may have a core portion composed of elemental iron, cobalt, or nickel and a surface portion composed of a passivating layer, such as a metal oxide or a noble metal coating, such as a layer of gold, platinum, palladium, or silver. In other embodiments, metal oxide magnetic nanoparticles or aggregates thereof are coated with a layer of a noble metal coating. The noble metal coating may, for example, reduce the number of charges on the magnetic nanoparticle surface, which may beneficially increase dispersibility in solution and better control the size of the BNCs. The noble metal coating protects the magnetic nanoparticles against oxidation, solubilization by leaching or by chelation when chelating organic acids, such as citrate, malonate, or tartrate, are used in the biochemical reactions or processes. The passivating layer can have any suitable thickness, and particularly, at least, up to, or less than, about for example, 0.1 nm, 0.2 nm, 0.3 nm, 0.4 nm, 0.5 nm, 0.6 nm, 0.7 nm, 0.8 nm, 0.9 nm, 1 nm, 2 nm, 3 nm, 4 nm, 5 nm, 6 nm, 7 nm, 8 nm, 9 nm, or 10 nm, or a thickness in a range bounded by any two of these values.

Magnetic materials useful for the invention are well-known in the art. Non-limiting examples comprise ferromagnetic and ferromagnetic materials including ores such as iron ore (magnetite or lodestone), cobalt, and nickel. In other embodiments, rare earth magnets are used. Non-limiting examples include neodymium, gadolinium, sysprosium, samarium-cobalt, neodymium-iron-boron, and the like. In yet further embodiments, the magnets comprise composite materials. Non-limiting examples include ceramic, ferrite, and alnico magnets. In preferred embodiments, the magnetic nanoparticles have an iron oxide composition. The iron oxide composition can be any of the magnetic or superparamagnetic iron oxide compositions known in the art, e.g., magnetite (FesO/O, hematite (α-Fe2θ 3), maghemite (γ-Fe2C>3), or a spinel ferrite according to the formula AB₂O₄, wherein A is a divalent metal (e.g., Xn²⁺, Ni²⁺, Mn²⁺, Co²⁺, Ba²⁺, Sr²⁺, or combination thereof) and B is a trivalent metal (e.g., Fe³⁺, Cr³⁺, or combination thereof).

In some embodiments, the BNC's are formed by exploiting the instability of superparamagnetic NPs. The Point of Zero Charges (PZC) of magnetite is pH 7.9, around which magnetic NPs cannot repel each other and cluster readily. NPs are positively charged below the PZC and negatively charged above it. Cluster formation may be driven by electrostatic Interactions. The opposite electrostatic charges at the surface of the enzymes from charged amino acids can compensate the surface charge of the NPs. Enzymes can be assimilated to poly-anions or poly-cations that neutralize the charge of multiple NPs. Each enzyme has its own isoelectric point (pI) and surface composition of charged amino acids that will trigger the aggregation of nanoparticles. The enzymes may then be entrapped and stabilized in mesoporous clusters. Initial NP and enzyme concentrations, pH and ionic strength are the main parameters controlling the aggregation rate and final cluster size. The size of the clusters greatly influences the efficacy of the reaction because of mass transport limitations of the substrates and products in-and-out of the clusters. They can be tuned from 100nm to 10µm clusters to control the enzyme loading and the substrate diffusion rates.

Entrapped enzymes are referred to Level 1. "Locked" clusters in rigid scaffolds may result from templating them onto or within bigger or more stable magnetic or polymeric scaffolds, referred as Level 2. This prevents over-aggregation and adds mass magnetization for ease of capture by external magnets.

In particular embodiments, the above mesoporous aggregates of magnetic nanoparticles (BNCs) are incorporated into a continuous macroporous scaffold to form a hierarchical catalyst assembly with first and second levels of assembly. The first level of assembly is found in the BNCs. The second level of assembly is found in the incorporation of the BNCs into the continuous macroporous scaffold. In some embodiments, the level 2 assembly is magnetic.

The term "continuous" as used herein for the macroporous magnetic scaffold, indicates a material that is not a particulate assembly, *i.e.,* is not constructed of particles or discrete objects assembled with each other to form a macroscopic structure. In contrast to a particulate assembly, the continuous structure is substantially seamless and uniform around macropores that periodically interrupt the seamless and uniform structure. The macropores in the continuous scaffold are, thus, not interstitial spaces between agglomerated particles. Nevertheless, the continuous scaffold can be constructed of an assembly or aggregation of smaller primary continuous scaffolds, as long as the assembly or aggregation of primary continuous scaffolds does not include macropores (*e.g.,* greater than about 50 nm and up to about 100) formed by interstitial spaces between primary continuous scaffolds. Particularly in the case of inorganic materials such as ceramics or elemental materials, the continuous scaffold may or may not also include crystalline domains or phase boundaries.

In particular embodiments, the above mesoporous aggregates of magnetic nanoparticles (BNCs) are incorporated into a continuous macroporous scaffold to form a hierarchical catalyst assembly with first and second levels of assembly. The first level of assembly is found in the BNCs. The second level of assembly is found in the incorporation of the BNCs into the continuous macroporous scaffold. The overall hierarchical catalyst assembly is magnetic by at least the presence of the BNCs.

The macroporous scaffold contains macropores (*i.e.,* pores of a macroscale size) having a size greater than 50 nm. In different embodiments, the macropores have a size of precisely, about, at least, above, up to, or less than, for example, 60 nm, 70 nm, 80 nm, 90 nm, 100 nm, 150 nm, 200 nm, 300 nm, 400 nm, 500 nm, 600 nm, 700 nm, 800 nm, 900 nm, 1 micron (1 µm), 1.2 µm, 1.5 µm, 2 µm, 3 µm, 4 µm, 5 µm, 10 µm, 20 µm, 30 µm, 40 µm, 50 µm, 60 µm, 70 µm, 80 µm, 90 µm, or 100 µm, or a size within a range bounded by any two of the foregoing exemplary sizes.

The macroporous scaffold can have any suitable size as long as it can accommodate macropores. In typical embodiments, the macroporous scaffold possesses at least one size dimension in the macroscale. The at least one macroscale dimension is above 50 nm, and can be any of the values provided above for the macropores, and in particular, a dimension of precisely, about, at least, above, up to, or less than, for example, 1 µm, 2 µm, 3 µm, 4 µm, 5 µm, 10 µm, 20 µm, 30 µm, 40 µm, 50 µm, 60 µm, 70 µm, 80 µm, 90 µm, 100 µm, 200 µm, 300 µm, 400 µm, 500 µm, 600 µm, 700 µm, 800 µm, 900 µm, 1 mm, 2 mm, 5 mm, or 1 cm, or a size within a range bounded by any two of the foregoing exemplary sizes. Where only one or two of the size dimensions are in the macroscale, the remaining one or two dimensions can be in the nanoscale, such as any of the values provided above for the magnetic nanoparticles (*e.g.,* independently, precisely, about, at least, above, up to, or less than, for example, 1, 2, 3, 4, 5, 10, 20, 30, 40, 50, 60, 70, 80, 90, or 100 nm, or a value within a range bounded by any two of the foregoing values). In some embodiments, at least two or all of the size dimensions of the macroporous scaffold is in the macroscale.

In a first set of embodiments, the continuous macroporous scaffold in which the BNCs are incorporated is magnetic, *i.e.,* even in the absence of the BNCs. The continuous macroporous scaffold can be magnetic by, for example, being composed of a magnetic polymer composition. An example of a magnetic polymer is PANiCNQ, which is a combination of tetracyanoquinodimethane (TCNQ) and the emeraldine-based form of polyaniline (PANi), as well known in the art. Alternatively, or in addition, the continuous macroporous scaffold can be magnetic by having embedded therein magnetic particles not belonging to the BNCs. The magnetic particles not belonging to the BNCs may be, for example, magnetic nano- or micro-particles not associated with an FRP enzyme or any enzyme. The magnetic microparticles may have a size or size distribution as provided above for the macropores, although independent of the macropore sizes. In particular embodiments, the magnetic microparticles have a size of about, precisely, or at least 20, 30, 40, 50, 60, 70, 80, 90, 100, 100, 200, 300, 400, 500, 600, 700, 800, 900, 1000 nm, or a size within a range bounded by any two of the foregoing exemplary sizes. In some embodiments, the continuous macroporous scaffold has embedded therein magnetic microparticles that are adsorbed to at least a portion of the BNCs, or wherein the magnetic microparticles are not associated with or adsorbed to the BNCs.

In a second set of embodiments, the continuous scaffold in which the BNCs are incorporated is non-magnetic. Nevertheless, the overall hierarchical catalyst assembly containing the non-magnetic scaffold remains magnetic by at least the presence of the BNCs incorporated therein.

In one embodiment, the continuous macroporous scaffold (or precursor thereof) has a polymeric composition. The polymeric composition can be any of the solid organic, inorganic, or hybrid organic-inorganic polymer compositions known in the art, and may be synthetic or a biopolymer that acts as a binder. Preferably, the polymeric macroporous scaffold does not dissolve or degrade in water or other medium in which the hierarchical catalyst is intended to be used. Some examples of synthetic organic polymers include the vinyl addition polymers (*e.g.,* polyethylene, polypropylene, polystyrene, polyacrylic acid or polyacrylate salt, polymethacrylic acid or polymethacrylate salt, poly(methylmethacrylate), polyvinyl acetate, polyvinyl alcohol, and the like), fluoropolymers (e.g., polyvinylfluoride, polyvinylidenefluoride, polytetrafluoroethylene, and the like), the epoxides (e.g., phenolic resins, resorcinol - formaldehyde resins), the polyamides, the polyurethanes, the polyesters, the polyimides, the polybenzimidazoles, and copolymers thereof. Some examples of biopolymers include the polysaccharides (e.g., cellulose, hemicellulose, xylan, chitosan, inulin, dextran, agarose, and alginic acid), polylactic acid, and polyglycolic acid. In the particular case of cellulose, the cellulose may be microbial- or algae-derived cellulose. Some examples of inorganic or hybrid organic-inorganic polymers include the polysiloxanes (e.g., as prepared by sol gel synthesis, such as polydimethylsiloxane) and polyphosphazenes. In some embodiments, any one or more classes or specific types of polymer compositions provided above are excluded as macroporous scaffolds.

In another embodiment, the continuous macroporous scaffold (or precursor thereof) has a non-polymeric composition. The non-polymeric composition can have, for example, a ceramic or elemental composition. The ceramic composition may be crystalline, polycrystalline, or amorphous, and may have any of the compositions known in the art, including oxide compositions (e.g., alumina, beryllia, ceria, yttria, or zirconia) and non-oxide compositions (e.g., carbide, silicide, nitride, boride, or sulfide compositions). The elemental composition may also be crystalline, polycrystalline, or amorphous, and may have any suitable elemental composition, such as carbon, aluminum, or silicon.

In other embodiments, the BNCs reside in a non-continuous macroporous support containing (or constructed of) an assembly (i.e., aggregation) of Magnetic Microparticles (MMPs) that includes macropores as interstitial spaces between the magnetic microparticles. The magnetic microparticles are typically ferromagnetic and can be made of magnetite or other ferromagnetic materials. The BNCs are embedded in at least a portion of the macropores of the aggregation of magnetic microparticles and may also reside on the surface of the magnetic microparticles. The BNCs can associate with the surface of the magnetic microparticles by magnetic interaction. The magnetic microparticles may or may not be coated with a metal oxide or noble metal coating layer. In some embodiments, the BNC-MMP assembly is incorporated (i.e., embedded) into a continuous macroporous scaffold, as described above, to provide a hierarchical catalyst assembly.

In some embodiments, the scaffolds comprise cross-linked water-insoluble polymers and an approximately uniform distribution of embedded magnetic microparticles (MMP). The cross-linked polymer comprises polyvinyl alcohol (PVA) and optionally additional polymeric materials. The scaffolds may take any shape by using a cast during preparation of the scaffolds. Alternatively, the scaffolds may be ground to microparticles for use in biocatalyst reactions. Alternatively, the scaffolds may be shaped as beads for use in biocatalyst reactions. Alternatively, the scaffolds may be monoliths. Methods for preparing and using the scaffolds are also provided.

In other embodiments, the magnetic macroporous polymeric hybrid scaffold comprises a cross-linked water-insoluble polymer and an approximately uniform distribution of embedded magnetic microparticles (MMP). The polymer comprises at least polyvinyl alcohol (PVA), has MMPs of about 50-500 nm in size, pores of about 1 to about 50 µm in size, about 20% to 95% w/w MMP, wherein the scaffold comprises an effective surface area for incorporating bionanocatalysts (BNC) that is about total 1-15 m²/g; wherein the total effective surface area for incorporating the enzymes is about 50 to 200 m²/g; wherein the scaffold has a bulk density of between about 0.01 and about 10 g/ml.; and wherein the scaffold has a mass magnetic susceptibility of about 1.0×10⁻³ to about 1×10⁻⁴ m³ kg⁻¹. In a preferred embodiment, the magnetic macroporous polymeric hybrid scaffold comprises a contact angle for the scaffold with water that is about 0-90 degrees. Details of the macroporous polymeric hybrid scaffold embodiments are taught in WO 2017/180383.

The individual magnetic nanoparticles or aggregates thereof or BNCs thereof possess any suitable degree of magnetism. For example, the magnetic nanoparticles, BNCs, or BNC scaffold assemblies can possess a saturated magnetization (Ms) of at least or up to about 5, 10, 15, 20, 25, 30, 40, 45, 50, 60, 70, 80, 90, or 100 emu/g. The magnetic nanoparticles, BNCs, or BNC-scaffold assemblies preferably possess a remanent magnetization (Mr) of no more than (i.e., up to) or less than 5 emu/g, and more preferably, up to or less than 4 emu/g, 3 emu/g, 2 emu/g, 1 emu/g, 0.5 emu/g, or 0.1 emu/g. The surface magnetic field of the magnetic nanoparticles, BNCs, or BNC-scaffold assemblies can be about or at least, for example, about 0.5, 1, 5, 10, 50, 100, 200, 300, 400, 500, 600, 700, 800, 900, or 1000 Gauss (G), or a magnetic field within a range bounded by any two of the foregoing values. If microparticles are included, the microparticles may also possess any of the above magnetic strengths.

The magnetic nanoparticles or aggregates thereof can be made to adsorb a suitable amount of enzyme, up to or below a saturation level, depending on the application, to produce the resulting BNC. In different embodiments, the magnetic nanoparticles or aggregates thereof may adsorb about, at least, up to, or less than, for example, 1, 5, 10, 15, 20, 25, or 30 pmol/m2 of enzyme. Alternatively, the magnetic nanoparticles or aggregates thereof may adsorb an amount of enzyme that is about, at least, up to, or less than, for example, about 10%, 20%, 30%, 40%, 50%, 60%, 70%, 80%, 90%, or 100% of a saturation level.

The magnetic nanoparticles or aggregates thereof or BNCs thereof possess any suitable pore volume. For example, the magnetic nanoparticles or aggregates thereof can possess a pore volume of about, at least, up to, or less than, for example, about 0.01, 0.05, 0.1, 0.15, 0. 2, 0.25, 0.3, 0.35, 0.4, 0.45, 0.5, 0.55, 0.6, 0.65, 0.7, 0.75, 0.8, 0.85, 0.9, 0.95, or 1 cm3/g, or a pore volume within a range bounded by any two of the foregoing values.

The magnetic nanoparticles or aggregates thereof or BNCs thereof possess any suitable specific surface area. For example, the magnetic nanoparticles or aggregates thereof can have a specific surface area of about, at least, up to, or less than, for example, about 50, 60, 70, 80, 90, 100, 110, 120, 130, 140, 150, 160, 170, 180, 190, or 200 m2/g.

MNPs, their structures, organizations, suitable enzymes, and uses are described in WO2012122437, WO2014055853, Int'l Application Nos. PCT/US16/31419 and PCT/US17/26086, and US20180200701. Automated continuous production of BNCs is disclosed in US20180200701. Magnetically immobilized enzymes and cofactor systems are described in WO2018102319.

The present description provides BNCs having magnetically-entrapped monooxygenases (E.C.1.13). In one embodiment, the monooxygenase is P450 (EC_1.14.-.-). In a preferred embodiment, the monooxygenase is of human origin. (*See, e.g.,* https://www.ncbi.nlm.nih.gov/pmc/articles/PMC2884625/.) In another preferred embodiment, the monooxygenase is of bacterial origin. In other preferred embodiments, the monooxygenase is of algal, fungal, plant or animal origin.

In some embodiments, the P450 is Human. In other embodiments, the human P450 is in an insoluble form and is embedded in the membranes of small vesicular organelles. The organelles may contain other enzymes that work with or enhance the activity of the monooxygenases. In other embodiments, the P450 is in a supersome. (*See, e.g.,* Corning, https://www.corning.com/worldwide/en/products/life-sciences/products/adme-tox-research/recombinant-metabolic-enzymes.html.) In other embodiments, the P450 is in a bactosome. (*See, e.g.,* Cypex, http://www.cypex.co.uk/ ezcypbuf.htm.)

In some embodiments, the P450 monooxygenase comprises a P450 sequence that is of an origin selected from the group consisting of primate, mouse, rat, dog, cat, horse, cow, sheep, and goat, or derivatives thereof. In other embodiments, the P450 monooxygenase comprises a P450 sequence that is of an origin selected from the group consisting of insect, fish, fungus, yeast, protozoan, and plant.

Cytochrome p450s (CYPs) (EC 1.14.13.-) are a diverse family of NAPDH-dependent oxidative hemeproteins present in all organisms. These enzymes, with expression profiles differing between tissues, carry out the metabolism of xenobiotics, or non-endogenous chemicals. (Denisov et al., Chem. Rev. 105(6):2253-78 (2005).) CYPs generate metabolites with higher solubility than their parent compounds to facilitate clearance from the body. The substrate range of CYPs is broad and varies between isoforms, which are capable of performing hydroxylation, epoxidation, deamination, dealkylation, and dearylation reactions, among others.

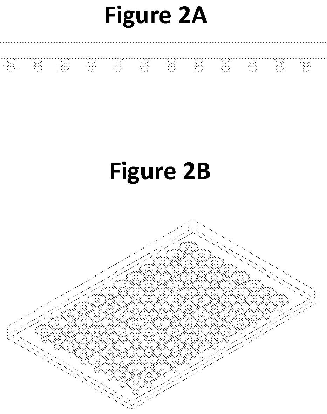

As part of safety due diligence for drugs, consumer products, and food additive development, tissue microsomes and recombinant CYPs are used to generate metabolites for evaluation of their toxicity. However, CYPs are notoriously challenging to use in industry as they often have low process stability and succumb to oxidative denaturation because of reactive oxygen species (ROS) formed as side products of CYP-mediated oxidations. Human CYPs are membrane bound and localize in the endoplasmic reticulum near cytochrome P450 reductase (CPR) and cytochrome b5, the latter sometimes improving CYP activity and the former required for activity. (Figure 2.)

The P450s used in the invention may perform aliphatic hydroxylations, aromatic hydroxylations, epoxidations, heteroatom dealkylation, alkyne oxygenations, heteroatom oxygenations, aromatic epoxidations and NIH-shift, dehalogenations, dehydrogenations, reduction and cleavage of esters.

The invention provides using other metabolic enzymes in the BNCs that produce metabolites in Phase I, II and III metabolism. Examples include UDP-glucuronosyl transferases, sulfotransferases, flavin-containing monooxygenases, monoamine oxidases, and carboxyesterases.

UDP-glucuronosyl transferases (UGT, EC2.4.1.17) enzymes catalyze the addition of a glucuronic acid moiety to xenobiotics. UGT's pathway is a major route of the human body's elimination of frequently prescribed drugs, xenobiotics, dietary substances, toxins, and endogenous toxins.

The superfamily of Sulfotransferases (E.C. 2.8.2.) are transferase enzymes that catalyze the transfer of a sulfo group from a donor molecule to an acceptor alcohol or amine. The most common sulfo group donor is 3'-phosphoadenosine-5'-phosphosulfate (PAPS). In the case of most xenobiotics and small endogenous substrates, sulfonation has generally been considered a detoxification pathway leading to more water-soluble products and thereby aiding their excretion via the kidneys or bile.

The flavin-containing monooxygenase (FMO, E.C. 1.14.13.8) enzymes perform the oxidation of xenobiotics to facilitate their excretion. These enzymes can oxidize a wide array of heteroatoms, particularly soft nucleophiles, such as amines, sulfides, and phosphites. This reaction requires dioxygen, an NADPH cofactor, and an FAD prosthetic group.

Monoamine oxidases (MAO, E.C. 1.4.3.4) catalyze the oxidative deamination of monoamines. Oxygen is used to remove an amine group from a molecule, resulting in the corresponding aldehyde and ammonia. MAO are well known enzymes in pharmacology, since they are the substrate for the action of a number of monoamine oxidase inhibitor drugs.

Carboxylesterases (E.C. 3.1.1.1) convert carboxylic esters and H₂O to alcohol and carboxylate. They are common in mammalian livers and participate in the metabolism of xenobiotics such as toxins or drugs; the resulting carboxylates are then conjugated by other enzymes to increase solubility and are eventually eliminated.

In some embodiments, the oxidoreductase used in the invention is a catalase. Catalases (EC. 1.11.1.6) are enzymes found in nearly all living organisms exposed to oxygen. They catalyze the decomposition of hydrogen peroxide (H₂O₂) to water and oxygen (O₂). They protect cells from oxidative damage by reactive oxygen species (ROS). Catalases have some of the highest turnover numbers of all enzymes; typically, one catalase molecule can convert millions of hydrogen peroxide molecules to water and oxygen each second. Catalases are tetramers of four polypeptide chains, each over 500 amino acids long. They contain four porphyrin heme (iron) groups that allow them to react with the hydrogen peroxide. Catalases are used in the food industry, *e.g.,* for removing hydrogen peroxide from milk prior to cheese production and for producing acidity regulators such as gluconic acid. Catalases are also used in the textile industry for removing hydrogen peroxide from fabrics.

In other embodiments, the oxidoreductase used in the invention is a superoxide dismutase (*e.g.,* EC 1.15.1.1). These are enzymes that alternately catalyzes the dismutation of the superoxide (O₂₋) radical into either ordinary molecular oxygen (O₂) or hydrogen peroxide (H₂O₂). Superoxide is produced as a by-product of oxygen metabolism and, if not regulated, causes oxidative damage. Hydrogen peroxide is also damaging but can be degraded by other enzymes such as catalase.

In other embodiments, the oxidoreductase is a glucose oxidase (*e.g.* Notatin, EC 1.1.3.4). It catalyzes the oxidation of glucose to hydrogen peroxide and D-glucono-δ-lactone. It is used, for example, to generate hydrogen peroxide as an oxidizing agent for hydrogen peroxide consuming enzymes such as peroxidase.

In other embodiments, the metabolic enzyme is a carboxylesterase (EC 3.1.1.1). Carboxylesterases are widely distributed in nature and are common in mammalian liver. Many participate in phase I metabolism of xenobiotics such as toxins or drugs; the resulting carboxylates are then conjugated by other enzymes to increase solubility and eventually excreted. The carboxylesterase family of evolutionarily related proteins (those with clear sequence homology to each other) includes a number of proteins with different substrate specificities, such as acetylcholinesterases.

The invention provides magnetically immobilized P450 catalytic systems for the production of chemical metabolites of P450. In some embodiments, enzyme stability or activity is maximized while reducing cofactor requirements. In other embodiments, the enzymes are immobilized on reusable magnetic carriers for metabolite manufacturing. In other embodiments, the magnetically immobilized P450 increases chemical manufacturing production capacity, enhances enzyme recovery, or decreases costs and environmental pollution. In other embodiments of the invention there is minimal to no loss in enzyme activity. In preferred embodiments, only about 1, 2, 3, 4, 5, 6, 7, 8, 9, 10, 11, 12, 13, 14, 15, 16-20, or 20-30% of the enzyme activity is lost. In other embodiments of the invention, there is an increase in enzyme activity and productivity. In other embodiments, one or more enzymes in addition to P450 are magnetically immobilized. This may facilitate the adoption of magnetic materials coupled with magnetic processes into existing manufacturing infrastructures or enable green chemistry methods.

The invention provides P450 metabolic enzymes/BNC-based biocatalytic syntheses that produce biologically relevant metabolites that are otherwise difficult to synthesize by traditional chemistry. In some embodiments, the invention mimics the diversity of metabolites that are produced by organisms upon exposure to xenobiotics. This is particularly relevant in the evaluation of drugs where oxidized metabolites can have adverse effects, or on the contrary, have higher pharmacological effects than a parent molecule from which it is derived. Here, metabolic profiling may increase the safety of new drugs. (*See* Metabolites in Safety Testing guideline by the U.S. Food and Drug Administration (FDA), http://www.fda.gov/downloads/Drugs/.../Guidances/ ucm079266.pdf.) Metabolic profiling of drugs and chemicals, in general, is limited by the difficulty of producing sufficient quantities of biologically relevant metabolites or by the difficulty of producing a diversity of metabolites in a high-throughput fashion.

The P450 cytochromes represent a gene superfamily of enzymes that are responsible for the oxidative metabolism of a wide variety of xenobiotics, including drugs. Wrighton and Stevens, Crit. Rev. Tox. 22(1):1-21 (1992); Kim et al., Xenobiotica 27(7):657-665 (1997): Tang, et al. J. Pharm. Exp. Therap., 293(2):453-459 (2000); Zhu et al., Drug Metabolism and Disposition 33(4):500-507 (2005); Trefzer et al. Appl. Environ. Microbiol. 73(13):4317-4325 (2007); Dresser et al. Clinical Pharmacokinetics 38(1):41-57 (2012). To generate drug metabolites in drug development, human liver microsomes, human-recombinant microsomes, or purified human-recombinant P450 monooxygenases are commercially available but typically suffer from process instability and poor activity levels. Iribarne, et al., Chem. Res. Tox. 9(2): p. 365-373 (1996); Yamazaki et al., Chem. Res. Tox. 11(6): p. 659-665 (1998); Joo et al., Nature, 399(6737):670-673 (1999).

The P450 BNCs described herein may be used, for example, in drug or specialty chemical manufacturing. In some embodiments, the manufactured compounds are small molecules. In other embodiments, the manufactured compounds are active pharmaceutical ingredients (API). In other embodiments, the manufactured compounds are active agricultural ingredients such as pesticides. In other embodiments, the manufactured compounds are active ingredients such as hormones and pheromones. In other embodiments, the manufactured compounds are flavors, fragrances and food coloring.

P450 enzymes are labile and notoriously difficult to use in biocatalytic reactions. They are, however, a major component of the metabolic pathway of drug and xenobiotic conversions and hence play a major role in the generation of drug metabolites. Human P450 have a broad range of substrates. For example, human CYP1A1 converts EROD to resofurin; human CYP1A2 converts phenacetin to acetaminophen and is also active on Clozapine, Olanzepine, Imipramine, Propranolol, and Theophylline; human CYP2A6 converts coumarin to 7-hydroxycoumarin; human CYP2B6 converts bupropion to hydroxybupropion and is also active on Cyclophosphamide, Efavirenz, Nevirapine, Artemisisin, Methadone, and Profofol; human CYP2C8 converts Paclitaxel to 6α-hydroxypaclitaxel; human CYP2C9 converts diclofenac to 4'-hydroxydiclofenac and is also active on Flurbiprofen, Ibuprofen, Naproxen, Phenytoin, Piroxicam Tolbutamide and Warfarin ; human CYP2C19 converts mephenytoin to 4'-hydroxyphenytoin and is also active on Amitriptyline, Cyclophosphamide, Diazepam, Imipramine, Omeprazole, and Phenytoin ; human CYP2D6 converts dextromethorphan to dextrorphan and is also active on Amitriptyline, Imipramine, Propranolol, Codeine, Dextromethorphan, Desipramine and Bufaralol ; human CYP2E1 is active on chlorzoxazone to 6-hydroxychlorzoxazone and also coverts Acetaminophen; human CYP2A4 converts midazolam to 1-hydroxymidazolam and is also active on Alprazolam, Carbamazepine, Testerone, Cyclosporine, Midazolam, Simvastatin, Triazolam and Diazepam.

Other metabolic enzymes such as human UGT, convert, for example, 7-hydroxycoumarin to 7-hydroxycoumarin glucuronide and human SULT converts 7-hydroxycoumarin to 7-hydroxycoumarin sulfate.

One difficulty in using monooxygenases in industrial processes is cofactor regeneration, and in particular, β-1,4-nicotinamide adenine dinucleotide phosphate (NADPH). NADPH is too expensive to be used stoichiometrically. Thus, in some embodiments, the invention provides cofactor regeneration compositions and methods to be used with the P450 BNCs. In preferred embodiments, the BNCs are used along with recycling enzymes. In more preferred embodiments, the recycling enzyme is Glucose Dehydrogenase (GDH). In other preferred embodiments, recycling enzymes such as GDH are co-immobilized with a P450.

The invention provides a process for the use of P450 metabolic enzymes magnetically-immobilized into BNCs. In some embodiments, machines provide magnetic mixing and capture P450.

The invention provides the use of enzymes that are expressed from a nucleic acid encoding enzyme polypeptides. In certain embodiments, the recombinant nucleic acids encoding an enzyme polypeptide may be operably linked to one or more regulatory nucleotide sequences in an expression construct. Regulatory nucleotide sequences will generally be appropriate for a host cell used for expression. Numerous types of appropriate expression vectors and suitable regulatory sequences are known in the art for a variety of host cells.

Typically, the one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are also contemplated. The promoters may be either naturally occurring promoters, or hybrid promoters that combine elements of more than one promoter. An expression construct may be present in a cell on an episome, such as a plasmid, or the expression construct may be inserted in a chromosome. In a specific embodiment, the expression vector includes a selectable marker gene to allow the selection of transformed host cells. Certain embodiments include an expression vector comprising a nucleotide sequence encoding an enzyme polypeptide operably linked to at least one regulatory sequence. Regulatory sequence for use herein include promoters, enhancers, and other expression control elements. In certain embodiments, an expression vector is designed considering the choice of the host cell to be transformed, the particular enzyme polypeptide desired to be expressed, the vector's copy number, the ability to control that copy number, or the expression of any other protein encoded by the vector, such as antibiotic markers.

Another aspect includes screening gene products of combinatorial libraries generated by the combinatorial mutagenesis of a nucleic acid described herein. Such screening methods include, for example, cloning the gene library into replicable expression vectors, transforming appropriate cells with the resulting library of vectors, and expressing the combinatorial genes under conditions to form such library. The screening methods optionally further comprise detecting a desired activity and isolating a product detected. Each of the illustrative assays described below are amenable to high-throughput analysis as necessary to screen large numbers of degenerate sequences created by combinatorial mutagenesis techniques.

Certain embodiments include expressing a nucleic acid in microorganisms. One embodiment includes expressing a nucleic acid in a bacterial system, for example, in *Bacillus brevis, Bacillus megaterium, Bacillus subtilis, Caulobacter crescentus, Escherichia coli* and their derivatives. Exemplary promoters include the l-arabinose inducible araBAD promoter (PBAD), the lac promoter, the l-rhamnose inducible rhaP BAD promoter, the T7 RNA polymerase promoter, the trc and tac promoter, the lambda phage promoter Pl, and the anhydrotetracycline-inducible tetA promoter/operator.

Other embodiments include expressing a nucleic acid in a yeast expression system. Exemplary promoters used in yeast vectors include the promoters for 3-phosphoglycerate kinase (Hitzeman et al., J. Biol. Chem. 255:2073 (1980)); other glycolytic enzymes (Hess et al., J. Adv. Enzyme Res. 7:149 (1968); Holland et al., Biochemistry 17:4900 (1978). Others promoters are from, e.g., enolase, glyceraldehyde-3-phosphate dehydrogenase, hexokinase, pyvurate decarboxylase, phosphofructokinase, glucose-6-phosphate isomerase, 3-phosphoglycerate mutase, pyruvate kinase, triosephosphate isomerase, phosphoglucose isomerase, glucokinase alcohol oxidase I (AOX1), alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter and termination sequences, with or without an origin of replication, is suitable. Certain yeast expression systems are commercially available, for example, from Clontech Laboratories, Inc. (Palo Alto, Calif., *e.g.* Pyex 4T family of vectors for S. cerevisiae), Invitrogen (Carlsbad, Calif., *e.g.* Ppicz series Easy Select Pichia Expression Kit) and Stratagene (La Jolla, Calif., *e.g.* ESP.TM. Yeast Protein Expression and Purification System for *S. pombe* and Pesc vectors for S. cerevisiae).

Other embodiments include expressing a nucleic acid in mammalian expression systems. Examples of suitable mammalian promoters include, for example, promoters from the following genes: ubiquitin/S27a promoter of the hamster (WO 97/15664), Simian vacuolating virus 40 (SV40) early promoter, adenovirus major late promoter, mouse metallothionein-I promoter, the long terminal repeat region of Rous Sarcoma Virus (RSV), mouse mammary tumor virus promoter (MMTV), Moloney murine leukemia virus Long Terminal repeat region, and the early promoter of human Cytomegalovirus (CMV). Examples of other heterologous mammalian promoters are the actin, immunoglobulin or heat shock promoter(s). In a specific embodiment, a yeast alcohol oxidase promoter is used.

In additional embodiments, promoters for use in mammalian host cells can be obtained from the genomes of viruses such as polyoma virus, fowlpox virus (UK 2,211,504 published 5 Jul. 1989), bovine papilloma virus, avian sarcoma virus, cytomegalovirus, a retrovirus, hepatitis-B virus and Simian Virus 40 (SV40). In further embodiments, heterologous mammalian promoters are used. Examples include the actin promoter, an immunoglobulin promoter, and heat-shock promoters. The early and late promoters of SV40 are conveniently obtained as an SV40 restriction fragment which also contains the SV40 viral origin of replication. Fiers et al., Nature 273: 113-120 (1978). The immediate early promoter of the human cytomegalovirus is conveniently obtained as a HindIII E restriction fragment. Greenaway, P. J. et al., Gene 18: 355-360 (1982).

Other embodiments include expressing a nucleic acid in insect cell expression systems. Eukaryotic expression systems employing insect cell hosts may rely on either plasmid or baculoviral expression systems. Typical insect host cells are derived from the fall army worm (*Spodoptera frugiperda*)*.* For expression of a foreign protein these cells are infected with a recombinant form of the baculovirus *Autographa californica* nuclear polyhedrosis virus which has the gene of interest expressed under the control of the viral polyhedron promoter. Other insects infected by this virus include a cell line known commercially as "High 5" (Invitrogen) which is derived from the cabbage looper (*Trichoplusia ni*)*.* Another baculovirus sometimes used is the Bombyx mori nuclear polyhedorsis virus which infect the silk worm (*Bombyx mori*)*.* Numerous baculovirus expression systems are commercially available, for example, from Thermo Fisher (Bac-N-Blue^{™}k or BAC-TO-BAC^{™} Systems), Clontech (BacPAK^{™} Baculovirus Expression System), Novagen (Bac Vector System^{™}), or others from Pharmingen or Quantum Biotechnologies. Another insect cell host is the common fruit fly, *Drosophila melanogaster,* for which a transient or stable plasmid based transfection kit is offered commercially by Thermo Fisher (The DES^{™} System).

In some embodiments, cells are transformed with vectors that express a nucleic acid described herein. Transformation techniques for inserting new genetic material into eukaryotic cells, including animal and plant cells, are well known. Viral vectors may be used for inserting expression cassettes into host cell genomes. Alternatively, the vectors may be transfected into the host cells. Transfection may be accomplished by calcium phosphate precipitation, electroporation, optical transfection, protoplast fusion, impalefection, and hydrodynamic delivery.

Certain embodiments include expressing a nucleic acid encoding an enzyme polypeptide in in mammalian cell lines, for example Chinese hamster ovary cells (CHO) and Vero cells. The method optionally further comprises recovering the enzyme polypeptide.

In some embodiments, the enzymes used in the invention are homologous to naturally-occurring enzymes. "Homologs" are bioactive molecules that are similar to a reference molecule at the nucleotide sequence, peptide sequence, functional, or structural level. Homologs may include sequence derivatives that share a certain percent identity with the reference sequence. Thus, in one embodiment, homologous or derivative sequences share at least a 70 percent sequence identity. In a specific embodiment, homologous or derivative sequences share at least an 80 or 85 percent sequence identity. In a specific embodiment, homologous or derivative sequences share at least a 90 percent sequence identity. In a specific embodiment, homologous or derivative sequences share at least a 95 percent sequence identity. In a more specific embodiment, homologous or derivative sequences share at least a 50, 55, 60, 65, 70, 75, 85, 86, 87, 88, 89, 90, 91, 92, 93, 94, 95, 96, 97, 98, or 99 percent sequence identity. Homologous or derivative nucleic acid sequences may also be defined by their ability to remain bound to a reference nucleic acid sequence under high stringency hybridization conditions. Homologs having a structural or functional similarity to a reference molecule may be chemical derivatives of the reference molecule. Methods of detecting, generating, and screening for structural and functional homologs as well as derivatives are known in the art.

The term percent "identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (*e.g.,* BLASTP and BLASTN or other algorithms available to persons of skill) or by visual inspection. Depending on the application, the percent "identity" can exist over a region of the sequence being compared, *e.g.,* over a functional domain, or, alternatively, exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel *et al., infra*)*.*

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

Another aspect described herein includes enzyme polypeptides that are synthesized in an *in vitro* synthesis reaction. In an example, the *in vitro* synthesis reaction is selected from the group consisting of cell-free protein synthesis, liquid phase protein synthesis, and solid phase protein synthesis as is well-known in the art.

In order that the invention described herein may be more fully understood, the following examples are set forth. It should be understood that these examples are for illustrative purposes only and are not to be construed as limiting this invention in any manner.

### EXAMPLES

### Example 1: Pin Manufacturing

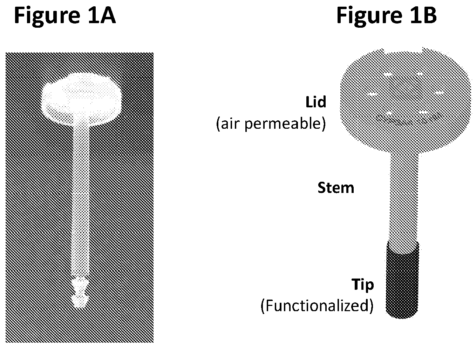

The material used to manufacture the pin was High Temperature Resin (289°C at 0.45 MPa, https://formlabs.com/store/us/form-2/materials/high-temp-resin/) purchased from Formlabs. The pin was modeled using a computer-aided design and exported to PreForm (Formlabs https://formlabs.com/tools/preform/) where the model is oriented and supported. The pin was 3D printed using a Form 2 (Formlabs https://formlabs.com/3d-printers/form-2/) printer and High Temp Resin. After printing, the pin was washed in two isopropyl alcohol baths for 5 minutes each. The pin was removed from the baths and air dried at room temperature. The pin was then placed in a post-curing chamber and exposed to 405 nm light for 60 minutes at 60° C. Finally, the supports were removed and the pin was wet sanded with water and sandpaper of 800 and 1200 grit until smooth. Printed pins are shown in Figure 1. A drawing of the microplate pin array is shown in Figure 2.

### Example 2: Functionalized Pin Coating Materials and Methods

The materials used for the fabrication of the functionalized pin coating were iron (II, III) oxide (magnetite, Sigma-Aldrich < 5 µm particles Cat# 310069-500G), carboxymethyl cellulose (CMC, Aqualon TM, CMC 7H3SXF PH, Ashland Cat# 426352), polyvinyl alcohol (PVA, 89-98K 99%, Aldrich Cat# 341584-500G), nano-fibrillated cellulose (NFC, 3% w/w water dispersion, Process Development Center, The University of Maine), anhydrous citric acid (VWR), xanthan gum (Judee's Gluten Free, 100% pure), and milli-Q water.

For the pin coating preparation, nano-fibrillated cellulose (NFC) was dispersed in water and sonicated at 40% amplitude for several seconds in an ice bath. Thereafter citric acid was added to the NFC dispersion and allowed to dissolve under continuous stirring for several minutes. Several milliliters of polyvinyl alcohol (PVA) and carboxymethyl cellulose (CMC) aqueous solutions were added while stirring the mixture. When the dispersion looked homogeneous, iron (II, III) oxide particles were added and the mixture was vigorously agitated. Finally, the mixture was sonicated at 35% amplitude for several seconds in an ice bath. The produced material was poured in silicone molds with the pins and frozen at the temperature range of -20° to -196° C, then lyophilized at -12° C and below 100 mTorr until the material was dried. After freeze drying the coated pins were crosslinked at 130° C for several minutes in an oven and the unreacted citric acid was removed by rinsing the material several times with water.

### Example 3: Pin Coating Formulation

A dispersion of NFC was produced by adding 1 g of a 3% w/w NFC dispersion into 6.3 mL of water through sonication at 40% amplitude for 1 min with a pulse of 30 seconds on and 10 seconds off. Then 80 mg of citric acid were added to the dispersion until dissolved at 500 RPM. This was followed by the addition of 2.7 mL of CMC 2% w/w and 1.37 mL of PVA 10% w/w aqueous solutions during agitation at 700 RPM. Then 823 mg of magnetite was added to the previous dispersion, agitated at 800 RPM for 2 min, then sonicated at 35% with a pulse of 6 seconds on and 2 seconds off for 1 min in an ice bath.

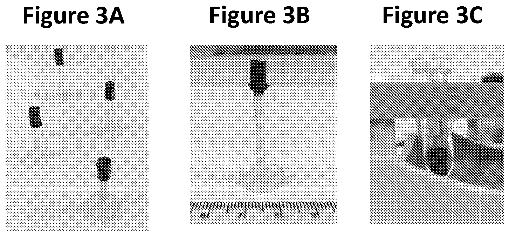

In another formulation, 1 g of NFC was dispersed in 17.41 mL of a xanthan gum 0.5% w/w water solution and 700 µL of water then sonicated as above. This was followed by the addition of 80 mg of citric acid. Thereafter, 2.7 mL of CMC 2% w/w and 700 µL of PVA 15% v/v were added during agitation at 700 RPM. When the dispersion looked homogeneous, 823 mg of magnetite were added and agitated at 800 RPM followed by sonication as above. Figure 3 shows some examples of the functionalized coated pins.

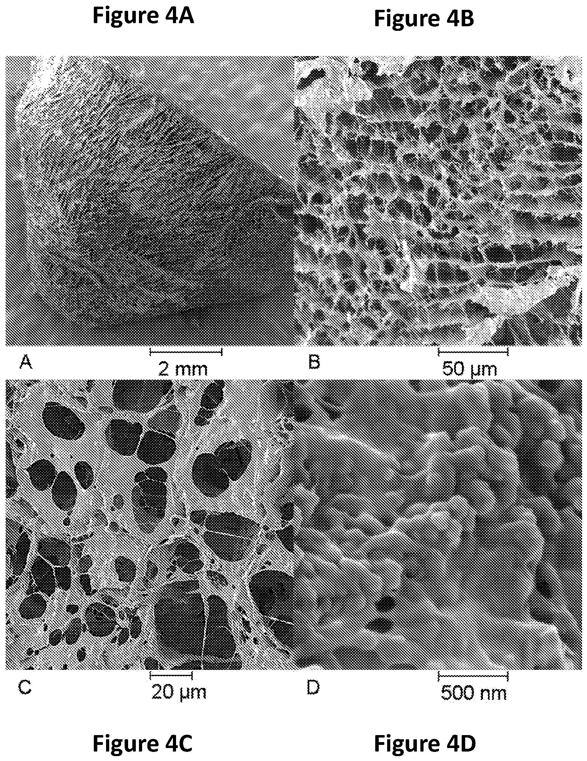

The microstructural features of the coating material were observed under a field emission scanning electron microscope (FESEM, Tescan Mira3) after being sputter coated (7 nm thickness) with a gold-palladium target. The SEM images of the crosslinked coating material are shown in Figure 4. In Figure 4A, the overall structure of a diamond shaped material is observed. Figure 4B shows a higher resolution image of the side surface which is the most dominant structure in the material.

### Example 4: Storage and Stability of immobilized CYP3A4

Immobilized CYP3A4 was stable when stored over seven days. A fluorometric surrogate substrate assay was developed to assess the activity of free and immobilized CYP3A4. CYP3A4 microsomes were purchased from Corning (cat. 456202: Human CYP3A4 + P450 Reductase + Cytochrome b₅ SUPERSOMES^{™}). Tris buffers were prepared from 1 M Tris pH 7.5 (KP BioMedical). All water was obtained from a BarnStead Nanopure water purifier (Thermo Scientific, 18.5 MOhm-cm). Trehalose (D-(+)-Trehalose, Dihydrate) and Sucrose were purchased from Fisher Scientific. Magnetite powder (Iron (II/III) oxide powder < 5 µm, 95%) was purchased from Sigma Aldrich (St. Louis, MO, USA).

The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 500 µL reaction volumes at 37°C and mixed on a rotator for 1-18 hours. CYP3A4 reactions contained 6.25 nM (3.125 pmol) CYP3A4, 100 mM KHPO₄ pH 7.5, 2 µM 7-ER, 2.3 mM MgCl₂, and the cofactor regeneration system consisting of 1.3 mM NADP, 5 U/mL G6PDH, and 2.3 mM G6P. The product resorufin (RFN) was detected by fluorescence at 535/587 nm excitation/emission and quantitated by comparing the fluorescence of a standard curve of RFN.

The immobilized CYP powders were washed twice with 5mM Tris (pH 7.5) and suspended in 200 µl of 5 mM Tris pH 7.5, 100 mM trehalose. Samples were (A) stored at 4° C, (B) frozen and stored at -20°C, and (C) frozen and stored at -80°C. After 1 day, 3 days, and 7 days, samples were rapidly thawed and/or warmed to room temperature, and the cryoprotectant/lyoprotectant buffer was removed and samples were washed with 100 mM KHPO₄. The CYP3A4 activity was subsequently performed as described.

The CYP3A4 activity did not diminish over the course of 7 days. The activity of the frozen samples (B, C) were slightly lower than those stored at 4 °C (A).

### Example 5: Extended CYP3A4 Activity

Immobilized CYP3A4 activity was significantly extended in cell-free assays when compared to free CYP3A4. CYP3A4 microsomes (cat. 456202: Human CYP3A4 + P450 Reductase + Cytochrome b₅ SUPERSOMES^{™}) were purchased from Corning. HEPES buffer was purchased from Acros Organics (HEPES sodium salt, 99%). All water was obtained from a BarnStead Nanopure water purifier (Thermo Scientific, 18.5 MOhm-cm). Sucrose was purchased from Fisher Scientific and magnetite powder (Iron (II/III) oxide powder < 5 µm, 95%) was purchased from Sigma Aldrich (St. Louis, MO, USA). Superoxide dismutase (from bovine erythrocytes; MP Biomedicals); Catalase (from bovine liver; Sigma Aldrich Cat# SRE0041), G6DH (Glucose-6-phosphate dehydrogenase from *Leuconostoc mesenteroides*; Alfa Aesar Cat# J60117-4I), NADP (β-Nicotinamide adenine dinucleotide 2'-phosphate reduced tetrasodium salt hydrate, >97%; Sigma Aldrich), and HEPES (HEPES sodium salt, 99%, ACROS Organics) were formulated and stored at -20° C. Catalase was freshly prepared for each immobilization. Stock solutions of superoxide dismutase (500 U/ml in water), G6DH (500 U/ml in pH 8 water) were prepared and stored at -20° C. Stock solutions of NADP (26 mM in water pH 8) were prepared and stored at -80°C. A stock solution of 26 mM MgCl₂ was prepared from magnesium chloride hexahydrate (Macron Chemicals).

Iron oxide nanoparticles (NPs) were prepared as previously described in Corgie et al., Catalysis & Biocatalysis - Chemistry Today 34(5):15-20 (2016) They were stored under a N₂ sparged atmosphere at pH 11 at 4°C. NPs were ultrasonicated (Fisher Scientific) on the day of immobilization for 1 min at 40% Amplitude. Nanoparticle solutions (2x) of 500 and 2000 µg/ml were prepared from a 22 mg/ml stock solution with pH 11 water. 260 µl of cold NP solution was added rapidly to 260 µl of a cold enzyme solution containing: superoxide dismutase (10 U/ml), catalase (20 ug/ml; 40-100 U/ml), G6DH (20 U/ml), and NADP (26 mM) in pH 4 water. Within 1-2 minutes of addition, a solution of CYP3A4 (12.5 pmol/ml) in cold 50 mM HEPES pH 7.5 was added to the NP-enzyme mix, and incubated for 1 hour at 4°C. The immobilization yield was quantified by the Bradford method (Bradford reagent: Quick Start^{™} Bradford 1x Dye Reagent #5000205 from BioRad) by comparing against CYP3A4 enzyme standards.

A fluorometric surrogate substrate assay was developed to assess the activity of free and immobilized CYP3A4. The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 500 µL reaction volumes at 37°C and mixed on a rotator for 1-18h. CYP3A4 reactions contained 6.25 nM (3.125 pmol) CYP3A4, 100 mM KHPO₄ pH 7.5, 2 µM 7-ER, 2.3 mM MgCl₂, and 2.3 mM G6P but lacked the cofactor regeneration system (NADP, G6PDH). The product (RFN) was detected by fluorescence at 535/587 nm excitation/emission and quantitated by comparing the fluorescence of a standard curve of RFN.

An increase of 280% and 370% increase in conversion from 7-ER to resorufin was observed between the 1 hr and 18 hr reaction time. (Figure 2.)

These show that immobilized CYP3A4 demonstrated activity past 1 hour with a dramatic increase in conversion ranging from 2.7- to 3.7-fold. By comparison, the free CYP3A4 is almost fully inactivated by 1 hour. This shows that immobilized CYP3A4 can metabolize substrates over extended periods and produce low-clearance metabolites. Additionally, the cofactor NADP and the cofactor regenerating enzyme G6DH was successfully immobilized and functional as demonstrated by the activity in the absence of added NADP or G6DH in free solution.

### Example 6: Human Liver Microsome Immobilization

Human liver microsomes (Corning ^{©} UltraPool^{™} HLM 150 Cat# 452117) were purchased from Corning. Tris buffers were prepared from 1 M Tris pH 7.5 (KP BioMedical). All water was obtained from a BarnStead Nanopure water purifier (Thermo Scientific, 18.5 MOhm-cm). Sucrose was purchased from Fisher Scientific and magnetite powder (Iron (II/III) oxide powder < 5 µm, 95%) was purchased from Sigma Aldrich (St. Louis, MO, USA).

The HLM stock (20 mg/ml) was rapidly thawed in a 37°C bath and diluted in cold 50 mM Tris pH 7.5 supplemented with 25 mM sucrose to a final concentration of 125 µg/ml. 500 µl of HLM solution was added to 5 mg of magnetite powder and incubated for 1 hour at 4°C. The immobilization yield was quantified by the Bradford method (Bradford reagent: Quick Start^{™} Bradford 1x Dye Reagent #5000205 from BioRad).

A fluorometric surrogate substrate assay was developed to assess the activity of free and immobilized HLM. The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 500 µL reaction volumes at 37°C and mixed on a rotator for 1 hour. HLM reactions contained 100 mM KHPO₄ pH 7.5, 2 µM 7-ER, 2.3 mM MgCl₂, and the cofactor regeneration system consisting of 1.3 mM NADP, 5 U/mL G6PDH, and 2.3 mM G6P. The product (RFN) was detected by fluorescence at 535/587 nm excitation/emission and quantitated by comparing the fluorescence to a standard curve of RFN.

The immobilization yield of HLM was determined in triplicate to be 96 +/-2.9%. The activity relative to free HLM at the same concentration was determined to be 55 +/- 4% (Figure 3).

The pooled human liver microsomes were almost fully immobilized and its CYP3A4 activity was shown by comparing it to the free HLM reaction. This demonstrates that the broad applicability of immobilizing CYP microsomes, including other microsomes (*e.g.* CYP2D6, CYP1A2, CYP2C9, UGT1A1), or combinations thereof.

### Example 7: Microsome Immobilization on Pin devices

An Enzyme Pin device was constructed by casting a suspension of PVA, CMC and citric acid (7% w/v) in a thin-walled cast in the shape of rounded pin and freezing it at -80°C as described in example 3. The pins were lyophilized for 3 days at -12°C and <100 mTorr, and immediately crosslinked by curing in the oven for 1 hour at 160°C.

CYP3A4 microsomes (cat. 456202: Human CYP3A4 + P450 Reductase + Cytochrome b₅ SUPERSOMES^{™}) were purchased from Corning. Tris buffer (pH 7.5, 1 M stock) was purchased KP BioMedicals. All water was obtained from a BarnStead Nanopure water purifier (Thermo Scientific, 18.5 MOhm-cm). Sucrose was purchased from Fisher Scientific and magnetite powder (Iron (II/III) oxide powder < 5 µm, 95%) was purchased from Sigma Aldrich (St. Louis, MO, USA). Superoxide dismutase (from bovine erythrocytes; MP Biomedicals); Catalase (from bovine liver; Aldrich), G6DH (Glucose-6-phosphate dehydrogenase from *Leuconostoc mesenteroides*; Alfa Aesar), NADP (β-Nicotinamide adenine dinucleotide 2'-phosphate reduced tetrasodium salt hydrate, >97%; Sigma Aldrich), HEPES (HEPES sodium salt, 99%, ACROS Organics) were formulated and stored at -20°C. Catalase was freshly prepared for each immobilization. Stock solutions of superoxide dismutase (500 U/ml in water), G6DH (500 U/ml in pH 8 water) were prepared and stored at -20°C. Stock solutions of NADP (26 mM in water pH 8) were prepared at stored at -80°C. A stock solution of 26 mM MgCl₂ was prepared from magnesium chloride hexahydrate (Macron Chemicals).

Iron oxide nanoparticles (NPs) were prepared as previously described and stored under a N₂ sparged atmosphere at pH 11 at 4°C. NPs were ultrasonicated (Fisher Scientific) on the day of immobilization for 1 min at 40% Amplitude.

Pin immobilization: A nanoparticle solution of 2500 µg/ml was prepared from a 22 mg/ml stock solution with pH 11 water. 100 µl of the cold NP solution (2500 µg/ml) was added rapidly to 100 µl of the enzyme solution containing superoxide dismutase (50 U/ml), catalase (100 µg/ml; 200-500 U/ml), G6DH (100 U/ml), and NADP (10 mM) in pH 4 water. Within 1-2 minutes of addition, 25 µl of the solution was added to the pin causing it to rapidly absorb the solution. The pin was incubated for 1 hour at 25°C before adding 25 µl of CYP3A4 (125 pmol/ml in 50 mM Tris pH 7.5, 25 mM Sucrose). After 1 hour of incubation at 4°C, the pin was immersed in 250 µl of water and the immobilization yield was quantified by the Bradford method (Bradford reagent: Quick Start^{™} Bradford 1x Dye Reagent #5000205 from BioRad) by comparing it to CYP3A4 enzyme standards.

CYP3A4 Activity: A fluorometric surrogate substrate assay was developed to assess the activity of free and immobilized HLM. The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 200 µL reaction volumes at 37°C and rotated on an orbital shaker at 500 rpm for 1 hour. CYP3A4 reactions contained 100 mM KHPO₄ pH 7.5, 2 µM 7-ER, 2.3 mM MgCl₂, and the cofactor regeneration system consisting of 1.3 mM NADP, 5 U/mL G6PDH, and 2.3 mM G6P. The product (RFN) was detected by fluorescence at 535/587 nm excitation/emission and quantitated by comparing the fluorescence to a standard curve of RFN.

The pin displayed a highly porous and spongy behavior and readily absorbed two 25 µl volumes of liquid. The immobilization yield of CYP3A4 and CAT/G6DH/SOD as determined by the Bradford method was 91.8 +/- 1.5 %, and the activity relative to free CYP3A4 was 29.5% +/- 3.8 %. Thus, both the cofactor and ROS recycling enzyme systems and CYP3A4 were successfully immobilized as a concentrated stock solution by drawing in the solutions in sequence by capillary forces.

### Example 8: Immobilization and Activity of CYP2B6 on Magnetite Powder

CYP2B6 is a member of the Cytochrome P450 family of enzymes. Enzymes in this family are monooxygenases involved in drug metabolism. Immobilized CYP2B6 activity was equal to or greater than the activity of the free enzyme for the same concentration of protein.

CYP2B6 enzyme (Coming^{®} Supersomes^{™} Human CYP2B6 + Oxidoreductase, Cat#456210) was purchased from Corning. Tris buffers were prepared from 1 M Tris pH 7.5 (KP BioMedical). All water was obtained from a BarnStead Nanopure water purifier (Thermo Scientific, 18.5 MOhm-cm). Magnetite powder (Iron (II/III) oxide powder < 10 µm) was purchased from Reade Advanced Materials.

The CYP2B6 stock (1 nmole/ml) was rapidly thawed in a 37°C bath and diluted in cold 50 mM Tris pH 7.0 to a final concentration of 4 pmol/ml. 500 µl of CYP2B6 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to CYP2B6 enzyme standards.

A luminescent substrate assay (Promega P450-Glo^{™} CYP2B6 Assay and Screening Systems) was used to measure the activity of the free and immobilized CYP2B6. The assay employs a luminogenic substrate derived from beetle luciferin. The derived substrate is not a substrate for luciferase but is converted by CYP2B6 to luciferin, which in turn reacts with luciferase to produce light that is directly proportional to the activity of CYP2B6 in the assay. Duplicate reactions were performed in 0.5 dram glass vials (VWR^{®} Vials, Borosilicate Glass, with Phenolic Screw Cap) with 500 µl reaction volume at room temperature with shaking for 2 hours. CYP2B6 reactions contained 100 mM KHPO₄ pH 7.5, 3 µM CYP2B6 luminescent substrate, and the cofactor regeneration system consisting of 1.3 mM NADP, 5 U/mL G6PDH, and 2.3 mM G6P. The product was detected by luminescence detection in a Synergy H1 Hybrid Multi-Mode Reader (BioTek).

The immobilization yield of CYP2B6 was determined in triplicate to be 85% +/- 1%. The activity relative to the free CYP2B6 enzyme was determined to be 114% +/-3%. Immobilized CYP2B6 had equal to or greater than activity of the free enzyme for the same concentration of protein. Immobilized CYP2B6 was easily recovered from the reaction vials, leaving only the reaction mix for analysis.

### Example 9: Storage and Stability of Immobilized CYP2B6

Immobilized CYP2B6 was stable when stored over fourteen days. CYP2B6 enzyme (Corning^{®} Supersomes^{™} Human CYP2B6 + Oxidoreductase, Cat#456210) was purchased from Corning. Tris buffers were prepared from 1 M Tris pH 7.5 (KP BioMedical). Sucrose was purchased from Fisher Scientific. All water was obtained from a BarnStead Nanopure water purifier (Thermo Scientific, 18.5 MOhm-cm). Magnetite powder (Iron (II/III) oxide powder < 10 µm) was Reade Advanced Materials.

The CYP2B6 stock (1 nmole/ml) was rapidly thawed in a 37°C bath and diluted in cold 50 mM Tris pH 7.0 to a final concentration of 4 pmol/ml. 500 µl of CYP2B6 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to CYP2B6 enzyme standards.

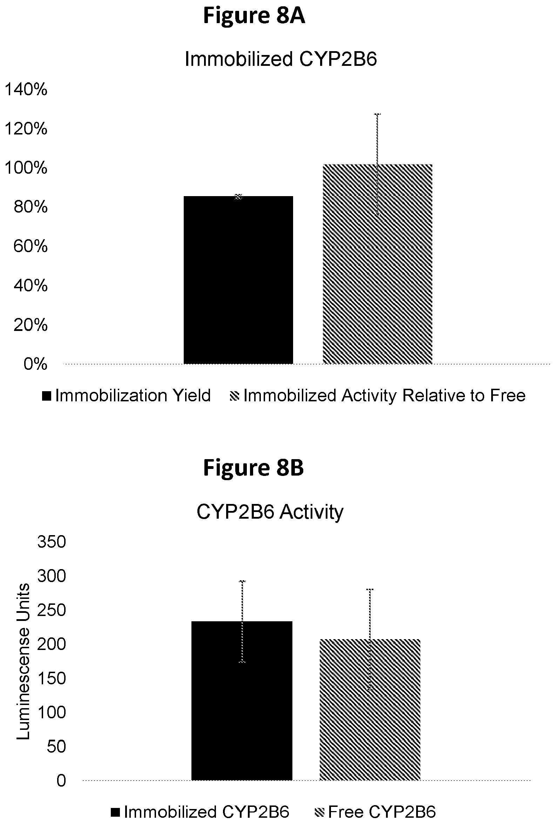

A luminescent substrate assay (Promega P450-Glo^{™} CYP2B6 Assay and Screening Systems) was used to measure the activity of the free and immobilized CYP2B6. The assay employs a luminogenic substrate derived from beetle luciferin. The derived substrate is not a substrate for luciferase but is converted by CYP2B6 to luciferin, which in turn reacts with luciferase to produce light that is directly proportional to the activity of CYP2B6 in the assay. Duplicate reactions were performed in 0.5 dram glass vials (VWR^{®} Vials, Borosilicate Glass, with Phenolic Screw Cap) with 500 µl reaction volume at room temperature with shaking for 2 hours. CYP2B6 reactions contained 100 mM KHPO₄ pH 7.5, 3 µM CYP2B6 luminescent substrate, and the cofactor regeneration system consisting of 1.3 mM NADP, 5 U/mL G6PDH, and 2.3 mM G6P. The product was detected by luminescence detection in a Synergy H1 Hybrid Multi-Mode Reader (BioTek) (Figure 8A).

The immobilized CYP2B6 powders were washed twice with 5mM Tris (pH 7.0) and suspended in 100 µl of 5 mM Tris pH 7.5, 100 mM sucrose. Samples were stored at -20°C. After 2 days, 7 days, and 14 days, samples were rapidly thawed in a 37°C bath, and the cryoprotectant buffer was removed and samples were washed with 100 mM KHPO₄. The CYP2B6 activity measurement was subsequently performed as described (Figure 8B).

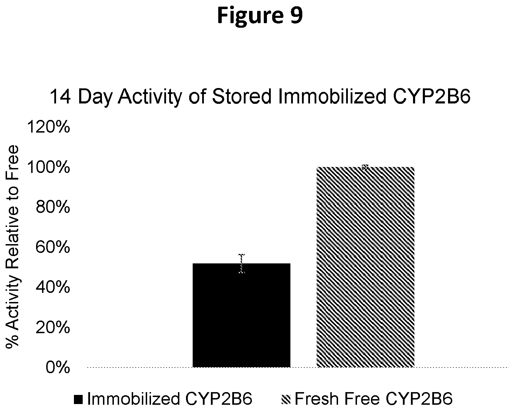

Immobilized CYP2B6 retained greater than or equal to 50% of the activity of fresh free CYP2B6 (Figure 9).

### Example 10: UGT1A6 Immobilized on Magnetite Powder

UGT1A6 is a UDP-glucuronosyltransferase, an enzyme of the glucuronidation pathway that transforms small lipophilic molecules into water-soluble, excretable metabolites. Immobilized UGT1A6 activity was equal to or greater than free UGT1A6 activity at the same protein concentration.

UGT1A6 enzyme (Coming^{®} Supersomes^{™} Human UGT1A6, Cat#456416) was purchased from Corning. Tris buffers were prepared from 1 M Tris pH 7.5 (KP BioMedical). All water was obtained from a BarnStead Nanopure water purifier (Thermo Scientific, 18.5 MOhm-cm). Sucrose was purchased from Fisher Scientific. Magnetite powder (Iron (II/III) oxide powder < 10 µm) was Reade Advanced Materials.

The UGT1A6 stock (5 mg/ mL) was rapidly thawed in a 37°C bath and diluted in cold 50 mM Tris pH 7.0 to a final concentration of 25 µg/ml. 500 µl of UGT1A6 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to UGT1A6 enzyme standards.

A fluorometric assay (BioVision UGT Activity Assay / Ligand Screening Kit, Cat# K692) was used to measure the activity of the free and immobilized UGT1A6. The assay utilizes a highly fluorescent UGT substrate and measures UGT activity by tracking the drop in fluorescence emission as the substrate is converted into a non-fluorescent glucuronide. UGT specific activity is calculated by comparing the fluorescence loss versus a control reaction performed in the absence of the required cofactor UDPGA. Duplicate reactions were performed in 0.5 dram glass vials (VWR^{®} Vials, Borosilicate Glass, with Phenolic Screw Cap) with 500 µl reaction volumes at 37°C with shaking for 2 hours. UGT1A6 reactions contained BioVision UGT Assay buffer with Alamethicin, BioVision UGT subtrate and the UDPGA cofactor. Control reactions contained BioVision UGT Assay buffer with Alamethicin, UGT substrate, and a volume of assay buffer instead of the UDPGA cofactor. The product was detected by fluorescence at Ex/Em = 415/502 nm and quantitated by comparing the drop in fluorescence to a standard curve of UGT substrate fluorescence.

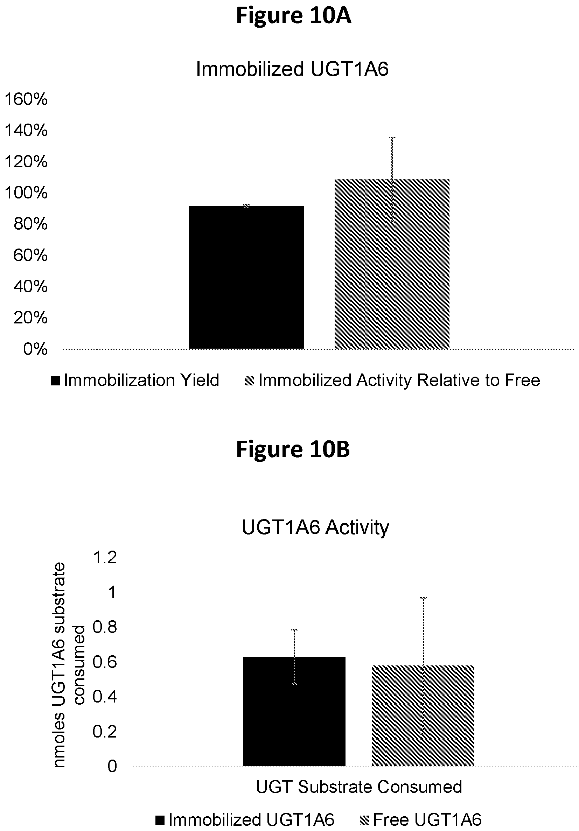

The immobilization yield of UGT1A6 was determined in triplicate to be 92% +/- 1% (Figure 10A). The activity relative to the free UGT1A6 enzyme at the same concentration was determined to be 109% +/- 27% (Figure 10A). Immobilized UGT1A6 consumed an average of 0.63±0.16 nmoles of BioVision UGT Activity Assay substrate and free UGT consumed an average of 0.58±0.39 nmoles of substrate (Figure 10B). Immobilized UGT1A6 was easily recovered from the reaction vials, leaving only the reaction mix for analysis.

### Example 11: Storage and Stability of Immobilized UGT1A6

UGT1A6 enzyme (Coming^{®} Supersomes^{™} Human UGT1A6, Cat#456416) was purchased from Corning. Tris buffers were prepared from 1 M Tris pH 7.5 (KP BioMedical). All water was obtained from a BarnStead Nanopure water purifier (Thermo Scientific, 18.5 MOhm-cm). Magnetite powder (Iron (II/III) oxide powder < 10 µm) was from Reade Advanced Materials.

The UGT1A6 stock (5 mg/ mL) was rapidly thawed in a 37°C bath and diluted in cold 50 mM Tris pH 7.0 to a final concentration of 25 µg/ml. 500 µl of UGT1A6 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to UGT1A6 enzyme standards.

A fluorometric assay (BioVision UGT Activity Assay / Ligand Screening Kit, Cat# K692) was used to measure the activity of the free and immobilized UGT1A6. The assay utilizes a highly fluorescent UGT substrate and measures UGT activity by tracking the drop in fluorescence emission as the substrate is converted into a non-fluorescent glucuronide. UGT specific activity is calculated by comparing the fluorescence loss versus a control reaction performed in the absence of the required cofactor UDPGA. Duplicate reactions were performed in 0.5 dram glass vials (VWR^{®} Vials, Borosilicate Glass, with Phenolic Screw Cap) with 500 µl reaction volumes at 37°C with shaking for 2 hours. UGT1A6 reactions contained BioVision UGT Assay buffer with Alamethicin, BioVision UGT subtrate and the UDPGA cofactor. Control reactions contained BioVision UGT Assay buffer with Alamethicin, UGT substrate, and a volume of assay buffer instead of the UDPGA cofactor. The product was detected by fluorescence at Ex/Em = 415/502 nm and quantitated by comparing the drop in fluorescence to a standard curve of UGT substrate fluorescence.

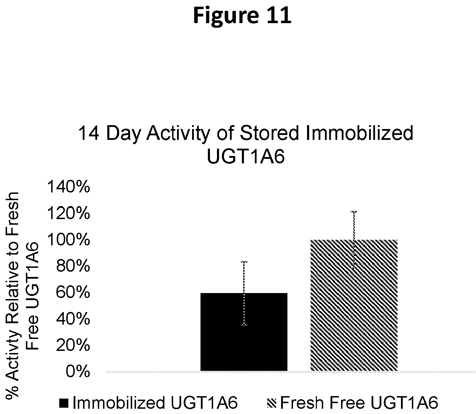

The immobilized UGT1A6 powders were washed twice with 5mM Tris (pH 7.0) and suspended in 100 µl of 5 mM Tris pH 7.5, 100 mM sucrose. Samples were stored at -20°C. After 1 day and 14 days, samples were rapidly thawed in a 37°C bath, and the cryoprotectant buffer was removed and the samples were washed with 100 mM KHPO₄. The UGT1A6 activity measurement was subsequently performed as described. Immobilized UGT1A6 retained greater than or equal to 50% of the activity of fresh free UGT1A6 (Figure 11).

### Example 12: CYP3A4 Immobilized on Magnetite Powders

Immobilization and Activity of CYP3A4 on Magnetite Powder. The immobilization reaction buffer pH was an important parameter in the immobilization reaction.

CYP3A4 enzyme (Corning^{®} Supersomes^{™} Human CYP3A4 + Oxidoreductase, Cat# 456207) was purchased from Corning. Tris buffers were prepared from 1 M Tris pH 7.5 (KP BioMedical). All water was obtained from a BarnStead Nanopure water purifier (Thermo Scientific, 18.5 MOhm-cm). Magnetite powder (Iron (II/III) oxide powder < 10 µm) was purchased from Reade Advanced Materials.

The CYP3A4 stock (2 nmole/ml) was rapidly thawed in a 37°C bath and diluted in cold 50 mM Tris pH 7.0, pH 7.5, or pH 8.0 to a final concentration of 6.25 pmol/ml. 500 µl of CYP3A4 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to CYP3A4 enzyme standards.

A fluorometric surrogate substrate assay was developed to assess the activity of free and immobilized CYP3A4. The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 500 µL reaction volumes at 37°C and mixed on a rotator for 1-18h. CYP3A4 reactions contained 6.25 nM (3.125 pmol) CYP3A4, 100 mM KHP04 pH 7.5, 2 µM 7-ER, 2.3 mM MgCl₂, and 2.3 mM G6P but lacked the cofactor regeneration system (NADP, G6PDH). The product (RFN) was detected by fluorescence at 535/587 nm excitation/emission and quantitated by comparing the fluorescence of a standard curve of RFN.

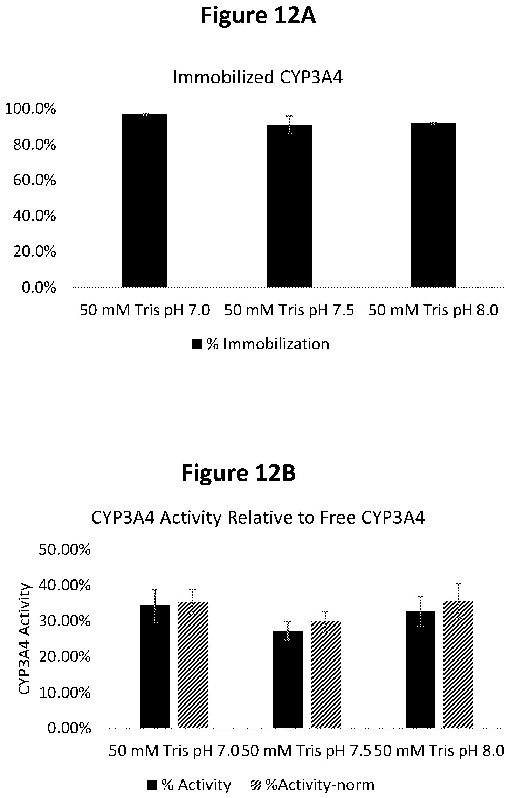

The immobilization yield of CYP3A4 was determined in duplicate to be 97%±0.4%, 91%±5%, and 92%±0.5% for immobilization buffer pH 7.0, pH 7.5, and pH 8.0 respectively (Figure 12A). The activity relative to the free CYP3A4 enzyme was determined to be 35%±3%, 30%±3%, and 36%±5% for immobilization buffer pH7.0, pH 7.5, and pH 8.0 respectively at the same concentration of protein (Figure 12B). Immobilized CYP3A4 was readily recovered from the reaction vials, leaving only the reaction mixture for analysis.

### Example 13: Storage and Stability of Immobilized CYP3A4

Immobilized CYP3A4 was stable when stored over fourteen days. CYP3A4 enzyme (Corning^{®} Supersomes^{™} Human CYP3A4 + Oxidoreductase, Cat# 456207) was purchased from Corning. Tris buffers were prepared from 1 M Tris pH 7.5 (KP BioMedical). All water was obtained from a BarnStead Nanopure water purifier (Thermo Scientific, 18.5 MOhm-cm). Magnetite powder (Iron (II/III) oxide powder < 10 µm) was purchased from Reade Advanced Materials.

The CYP3A4 stock (2 nmole/ml) was rapidly thawed in a 37°C bath and diluted in cold 50 mM Tris pH 7.0 to a final concentration of 6.25 pmol/ml. 500 µl of CYP3A4 solution was added to 5 mg of magnetite powder and incubated for 1 hour at 4°C with shaking. The immobilization yield was quantified by the Bradford method (Bradford reagent: Quick Start^{™} Bradford 1x Dye Reagent #5000205 from BioRad) by comparing it to CYP3A4 enzyme standards.

A fluorometric surrogate substrate assay was developed to assess the activity of free and immobilized CYP3A4. The assay measured dealkylation of 7-ethoxyresorufin (7-ER) to resorufin (RFN). 7-ER was purchased from BioVision (Cytochrome P450 3A4 (CYP3A4) Activity Assay Kit; K701200). Duplicate reactions were performed in 2 mL tubes with 500 µL reaction volumes at 37°C and mixed on a rotator for 1-18h. CYP3A4 reactions contained 6.25 nM (3.125 pmol) CYP3A4, 100 mM KHP04 pH 7.5, 2 µM 7-ER, 2.3 mM MgCl₂, and 2.3 mM G6P but lacked the cofactor regeneration system (NADP, G6PDH). The product (RFN) was detected by fluorescence at 535/587 nm excitation/emission and quantitated by comparing the fluorescence of a standard curve of RFN.

The immobilized CYP3A4 powders were washed twice with 5mM Tris (pH 7.0) and suspended in 100 µl of 5 mM Tris pH 7.5, 100 mM sucrose. Samples were stored at 1) -4°C, 2) -80°C, 3) -20°C and 4) freeze dried then stored at 4°C. After 1 day, 3 days, 7 days, and 14 days, samples were rapidly thawed in a 37°C bath, and the cryoprotectant buffer was removed and samples were washed with 100 mM KHPO₄. The CYP3A4 activity was subsequently measured as described above.

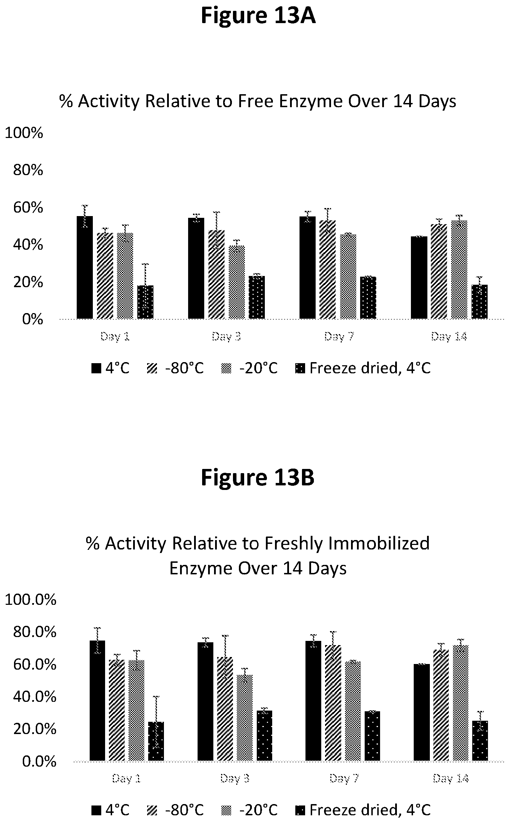

Immobilized CYP3A4 stored at 4°C retained 55%±6%, 54%±2%, 55%±3%, and 44%±0. 1% activity relative to free CYP3A4 over 1, 3, 7, and 14 days respectively. Immobilized CYP3A4 stored at 4°C retained 75%±8%, 74%±3%, 75%±4%, and 60%±0.1% relative to freshly immobilized CYP3A4 over 1, 3, 7, and 14 days respectively. Immobilized CYP3A4 stored at -80°C retained 46%±3%, 48%±10%, 53%±6%, and 51%±3% activity relative to free CYP3A4 over 1, 3, 7, and 14 days respectively. Immobilized CYP3A4 stored at -80°C retained 63%3%, 65%13%, 72%8%, and 69%4% relative to freshly immobilized CYP3A4 over 1, 3, 7, and 14 days respectively. Immobilized CYP3A4 stored at -20°C retained 46%±4%, 39%±3%, 46%±1%, and 53%±3% activity relative to free CYP3A4 over 1, 3, 7, and 14 days respectively. Immobilized CYP3A4 stored at -20°C retained 63%±6%, 53%±4%, 62%±1%, and 72%±4% relative to freshly immobilized CYP3A4 over 1, 3, 7, and 14 days respectively (Figure 13A). Immobilized CYP3A4 freeze dried and then stored at 4°C retained 18%±12%, 23%±1%, 23%±0%, and 19%±4% activity relative to free CYP3A4 over 1, 3, 7, and 14 days respectively. Immobilized CYP3A4 freeze dried and then stored at 4°C retained 25%±16%, 31%±2%, 31%±0.3%, and 25%±6% relative to freshly immobilized CYP3A4 over 1, 3, 7, and 14 days respectively (Figure 13B).

### Exemplary Sequences

**SEQ ID NO:1**
   **Cytochrome P450 3A4 isoform 1 [*Homo sapiens*]**
**SEQ ID NO:2**
   **Cytochrome P450 1A2 [*Homo sapiens*]** 
MALSQSVPFSATELLLASAIFCLVFWVLKGLRPRVPKGLKSPPEPWGWPLLGHVL
TLGKNPHLALSRMSQRYGDVLQIRIGSTPVLVLSRLDTIRQALVRQGDDFKGRPD
LYTSTLITDGQSLTFSTDSGPVWAARRRLAQNALNTFSIASDPASSSSCYLEEHVS
KEAKALISRLQELMAGPGHFDPYNQVVVSVANVIGAMCFGQHFPESSDEMLSLV
KNTHEFVETASSGNPLDFFPILRYLPNPALQRFKAFNQRFLWFLQKTVQEHYQDF
DKNSVRDITGALFKHSKKGPRASGNLIPQEKIVNLVNDIFGAGFDTVTTAISWSLM
YLVTKPEIQRKIQKELDTVIGRERRPRLSDRPQLPYLEAFILETFRHSSFLPFTIPHST
TRDTTLNGFYIPKKCCVFVNQWQVNHDPELWEDPSEFRPERFLTADGTAINKPLS
 
EKMMLFGMGKRRCIGEVLAKWEIFLFLAILLQQLEFSVPPGVKVDLTPIYGLTMK
HARCEHVQARLRFSIN
**SEQ ID NO:3**
   **CYP2D6 [*Homo sapiens*]**
**SEQ ID NO:4**
   **Cytochrome P450-2E1 [*Homo sapiens*]**
**SEQ ID NO:5**
   **Cytochrome P450-2E1 [*Homo sapiens*]**
**SEQ ID NO:6**
   **Cytochrome P450, family 2, subfamily C, polypeptide 9 [*Homo sapiens*]**

The foregoing description has been presented only for purposes of illustration and description. This description is not intended to limit the invention to the precise form disclosed. It is intended that the scope of the invention be defined by the claims appended hereto.

## Claims

1. A device for converting diffusible substrates into diffusible products, comprising microsomes and self-assembled mesoporous aggregates of magnetic nanoparticles, wherein a first enzyme requiring a diffusible cofactor having a first enzymatic activity is contained within said microsomes, wherein a second enzyme comprising a cofactor regeneration activity is magnetically-entrapped within said mesopores,
wherein said cofactor is utilized in said first enzymatic activity;
wherein said magnetic nanoparticles are associated with a macroporous scaffold;
wherein said microsomes are associated with said macroporous scaffold; and
wherein said macroporous scaffold comprising said magnetic nanoparticles is associated with a non-reactive portion operable for placing said macroporous scaffold into or removing it from a reaction solution;
a. optionally wherein one or more said enzymes are produced by recombinant DNA technology or are synthesized; or
b. optionally wherein said magnetic nanoparticles comprise human liver microsomes (HLM), enzymes from a human liver cytosol fraction (HLCF), or UGT1A6.

2. The device of claim 1, further comprising a functional portion for stably maintaining said microsomes, said magnetic nanoparticles, said first enzyme, said second enzyme, and said macroporous scaffold;
a. optionally wherein said functional portion comprises a buffer;
b. optionally wherein said functional portion comprises a substrate for said second enzyme;
c. optionally wherein said functional portion comprises said cofactor;
d. optionally wherein said functional portion is magnetic;
e. optionally wherein said co-factor is entrapped in said mesoporous aggregates of magnetic nanoparticles with said first and second enzymes; or
f. optionally wherein said macroporous scaffold is in the shape of a cylindrical pin, an orb, a bead, a capsule, a cube, a squared rod, a pyramid, a diamond, or is amorphous.

3. The device of either one of claims 1 or 2, wherein said non-reactive portion comprises metal, plastic, ceramic, a composite, or a combination thereof;
a. optionally wherein said non-reactive portion comprises a handle for manipulating said device;
b. optionally wherein said non-reactive portion is operable for handling by a robotic arm for high-throughput screening; or
c. optionally wherein said non-reactive portion allows for diffusion of gases.

4. The device of any one of claims 1-3, wherein said mesoporous aggregates of magnetic nanoparticles have an iron oxide composition;
a. optionally wherein said mesoporous aggregates of magnetic nanoparticles have a magnetic nanoparticle size distribution in which at least 90% of said magnetic nanoparticles have a size of at least 3 nm and up to 30 nm, and an aggregated particle size distribution in which at least 90% of said mesoporous aggregates of magnetic nanoparticles have a size of at least 10 nm and up to 500 nm;
b. optionally wherein said mesoporous aggregates of magnetic nanoparticles possess a saturated magnetization of at least 10 emu/g; or
c. optionally wherein said mesoporous aggregates of magnetic nanoparticles possess a remnant magnetization up to 5 emu/g.

5. The device of any one of claims 1-4, wherein said first and second enzymes are contained in said mesoporous aggregates of magnetic nanoparticles in up to 100% of saturation capacity; optionally wherein said first and second enzymes are physically inaccessible to microbes.

6. The device of any one of claims 1-5, wherein said first enzyme is an oxidative enzyme; optionally wherein said oxidative enzyme is a Flavin-containing oxygenase; wherein said composition further comprises a third enzyme having a co-factor reductase activity that is co-located with said first enzyme; optionally wherein said third enzyme is a cytochrome P450 reductase.

7. The device of claim 6, wherein said oxidative enzyme is a P450 monooxygenase;
a. optionally wherein a single protein comprises said P450 monooxygenase and said third enzyme;
b. optionally wherein said P450 monooxygenase is co-located with said third enzyme within a lipid membrane;
c. optionally wherein said P450 monooxygenase comprises a P450 sequence that is mammalian; optionally wherein said P450 monooxygenase comprises a P450 sequence that is human;
d. optionally wherein said P450 monooxygenase comprises CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1,CYP3A4, CYP3A5, CYP3A7, CYP3A43,CYP4A11, CYP4A22, CYP4B1, CYP4F2, CYP4F3, CYP4F8, CYP4F11, CYP4F12, CYP4F22, CYP4V2, CYP4X1, CYP4Z1,CYP5A1,CYP7A1, CYP7B1,CYP8A1, CYP8B1,CYP11A1, CYP11B1, CYP11B2, CYP17A1, CYP19A1, CYP20A1, CYP21A2, CYP24A1, CYP26A1, CYP26B1, CYP26C1, CYP27A1, CYP27B1, CYP27C1, CYP39A1, CYP46A1, or CYP51A1.

8. The device of any one of claims 1-7, wherein said second enzyme is selected from the group consisting of a carbonyl reductase, an aldehyde dehydrogenase, an aryl-alcohol dehydrogenase, an alcohol dehydrogenase, a pyruvate dehydrogenase, a D-1 xylose dehydrogenase, an oxoglutarate dehydrogenase, an isopropanol dehydrogenase, a glucose-6-phosphate dehydrogenase, a glucose dehydrogenase, a malate dehydrogenase, a formate dehydrogenase, a benzaldehyde dehydrogenase, a glutamate dehydrogenase, and an isocitrate dehydrogenase.

9. The device of any one of claims 1-8, wherein said cofactor is nicotinamide adenine dinucleotide + hydrogen (NADH), nicotinamide adenine dinucleotide phosphate + hydrogen (NADPH), Flavin adenine dinucleotide + hydrogen (FADH), or glutathione.

10. The device of any one of claims 1-9, wherein said first enzyme participates in phase I or wherein the device further comprises a third enzyme that participates in phase II metabolism.

11. The device of any one of claims 1-10, further comprising a fourth enzyme that reduces a reactive oxygen species (ROS); optionally wherein said fourth enzyme is a catalase, a superoxide dismutase (SOD), or a glutathione peroxidase/glutathione-disulfide reductase or a combination thereof.

12. The device of any one of claims 1-11, further comprising a fifth enzyme selected from the group consisting of a UDP-glucoronosyl transferase, a sulfotransferase, a monoamine oxidase, and a carboxylesterase.

13. The device of any one of claims 1-12, wherein said macroporous scaffold is a magnetic macroporous scaffold;
a. optionally wherein said macroporous magnetic scaffold is a polymeric hybrid scaffold comprising a cross-linked water-insoluble polymer and an approximately uniform distribution of embedded magnetic microparticles (MMP);
i. optionally wherein said magnetic macroporous polymeric hybrid scaffold comprises PVA and a polymer selected from the group consisting of CMC, alginate, HEC, EHEC; or
ii. optionally wherein said magnetic macroporous polymeric hybrid scaffold comprises a hydrophilic polymer; optionally wherein said hydrophilic polymer is xanthan gum.

14. A method of producing metabolites of a compound, comprising mixing said compound with a diffusible substrate in a reaction solution, positioning the device of any one of claims 1-13 such that said macroporous scaffold comprising said magnetic nanoparticles contacts said reaction solution, and measuring a product resulting from an enzymatic reaction in said reaction solution;
a. optionally further comprising the step of removing said macroporous scaffold comprising said microsomes and said magnetic nanoparticles from said reaction solution;
b. optionally wherein said method is incorporated into a high-throughput screening method for screening the toxicity of a plurality of compounds; or
c. optionally wherein said method is incorporated into a high-throughput screening method for screening metabolites from mixtures of metabolic enzymes.

15. A method of manufacturing the device of any one of claims 1-13, comprising magnetically entrapping said second enzyme in said mesoporous aggregates of magnetic nanoparticles, combining said aggregates with said second enzyme with said microsomes comprising said first enzyme, templating said aggregates and microsomes onto said macroporous scaffolds, and templating said scaffolds onto said non-reactive portion;
a. optionally wherein said aggregates further comprise a third enzyme having a co-factor reductase activity;
i. optionally wherein said aggregates further comprise a fourth enzyme that is a catalase, a superoxide dismutase (SOD), or a glutathione peroxidase/glutathione-disulfide reductase; or
ii. optionally wherein said aggregates further comprise a fifth enzyme that participates in phase II metabolism.

## Patentansprüche

1. Vorrichtung zur Umwandlung diffusionsfähiger Substrate in diffusionsfähige Produkte, umfassend Mikrosomen und selbstorganisierte mesoporöse Aggregate magnetischer Nanopartikel, wobei ein einen diffusionsfähigen Cofaktor benötigendes erstes Enzym mit einer ersten enzymatischen Aktivität in den Mikrosomen enthalten ist, wobei ein eine Cofaktorregenerationsaktivität umfassendes zweites Enzym magnetisch eingefangen in den Mesoporen vorliegt,
wobei der Cofaktor bei der ersten enzymatischen Aktivität genutzt wird;
wobei die magnetischen Nanopartikel mit einem makroporösen Gerüst assoziiert sind;
wobei die Mikrosomen mit dem makroporösen Gerüst assoziiert sind; und
wobei das die magnetischen Nanopartikel umfassende makroporöse Gerüst mit einem nicht reaktiven Teil assoziiert ist, der zum Einbringen des makroporösen Gerüsts in eine Reaktionslösung oder Entfernen daraus dient;
a. gegebenenfalls wobei eines oder mehrere der Enzyme mittels DNA-Rekombinationstechnologie erzeugt oder synthetisiert werden; oder
b. gegebenenfalls wobei die magnetischen Nanopartikel Mikrosomen aus menschlicher Leber (Human Liver Microsomes, HLM), Enzyme aus einer Cytosolfraktion von menschlicher Leber (Human Liver Cytosol Fraction, HLCF) oder UGT1A6 umfassen.

2. Vorrichtung nach Anspruch 1, ferner umfassend einen Funktionsteil zur stabilen Erhaltung der Mikrosomen, der magnetischen Nanopartikel, des ersten Enzyms, des zweiten Enzyms und des makroporösen Gerüsts;
a. gegebenenfalls wobei der Funktionsteil einen Puffer umfasst;
b. gegebenenfalls wobei der Funktionsteil ein Substrat für das zweite Enzym umfasst;
c. gegebenenfalls wobei der Funktionsteil den Cofaktor umfasst;
d. gegebenenfalls wobei der Funktionsteil magnetisch ist;
e. gegebenenfalls wobei der Cofaktor in den mesoporösen Aggregaten magnetischer Nanopartikel mit dem ersten und dem zweiten Enzym gefangen ist; oder
f. gegebenenfalls wobei das makroporöse Gerüst in Form eines zylindrischen Stiftes, einer Kugel, einer Perle, einer Kapsel, eines Würfels, eines vierkantigen Stäbchens, einer Pyramide, eines Diamanten oder amorph vorliegt.

3. Vorrichtung nach Anspruch 1 oder 2, wobei der nicht reaktive Teil Metall, Kunststoff, Keramik, einen Verbundstoff oder eine Kombination davon umfasst;
a. gegebenenfalls wobei der nicht reaktive Teil einen Griff zur Handhabung der Vorrichtung umfasst;
b. gegebenenfalls wobei der nicht reaktive Teil der Bedienung über einen Roboterarm für ein Screening mit hohem Durchsatz dient; oder
c. gegebenenfalls wobei der nicht reaktive Teil die Diffusion von Gasen ermöglicht.

4. Vorrichtung nach einem der Ansprüche 1-3, wobei die mesoporösen Aggregate magnetischer Nanopartikel eine Eisenoxidzusammensetzung aufweisen;
a. gegebenenfalls wobei die mesoporösen Aggregate magnetischer Nanopartikel eine Größenverteilung der magnetischen Nanopartikel, bei der wenigstens 90 % der magnetischen Nanopartikel eine Größe von wenigstens 3 nm und bis zu 30 nm aufweisen, und eine Größenverteilung der aggregierten Partikel, bei der wenigstens 90 % der mesoporösen Aggregate magnetischer Nanopartikel eine Größe von wenigstens 10 nm und bis zu 500 nm aufweisen, aufweisen;
b. gegebenenfalls wobei die mesoporösen Aggregate magnetischer Nanopartikel eine gesättigte Magnetisierung von wenigstens 10 emu/g besitzen; oder
b. gegebenenfalls wobei die mesoporösen Aggregate magnetischer Nanopartikel eine Restmagnetisierung von bis zu 5 emu/g besitzen.

5. Vorrichtung nach einem der Ansprüche 1-4, wobei das erste und das zweite Enzym in den mesoporösen Aggregaten magnetischer Nanopartikel bei bis zu 100 % Sättigungskapazität enthalten sind; gegebenenfalls wobei das erste und das zweite Enzym für Mikroben physikalisch nicht erreichbar sind.

6. Vorrichtung nach einem der Ansprüche 1-5, wobei es sich bei dem ersten Enzym um ein oxidatives Enzym handelt; gegebenenfalls wobei es sich bei dem oxidativen Enzym um eine flavinhaltige Oxygenase handelt; wobei die Zusammensetzung ferner ein drittes Enzym mit einer Cofaktor-Reduktase-Aktivität umfasst, das gemeinsam mit dem ersten Enzym lokalisiert ist; gegebenenfalls wobei es sich bei dem dritten Enzym um eine Cytochrom-P450-Reduktase handelt.

7. Vorrichtung nach Anspruch 6, wobei es sich bei dem oxidativen Enzym um eine P450-Monooxygenase handelt;
a. gegebenenfalls wobei ein einzelnes Protein die P450-Monooxygenase und das dritte Enzym umfasst;
b. gegebenenfalls wobei die P450-Monooxygenase gemeinsam mit dem dritten Enzym in einer Lipidmembran lokalisiert ist;
c. gegebenenfalls wobei die P450-Monooxygenase eine P450-Sequenz umfasst, die von einem Säuger stammt; gegebenenfalls wobei die P450-Monooxygenase eine P450-Sequenz umfasst, die vom Menschen stammt;
d. gegebenenfalls wobei die P450-Monooxygenase CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1,CYP3A4, CYP3A5, CYP3A7, CYP3A43,CYP4A11, CYP4A22, CYP4B1, CYP4F2, CYP4F3, CYP4F8, CYP4F11, CYP4F12, CYP4F22, CYP4V2, CYP4X1, CYP4Z1,CYP5A1,CYP7A1, CYP7B1,CYP8A1, CYP8B1,CYP11A1, CYP11B1, CYP11B2, CYP17A1, CYP19A1, CYP20A1, CYP21A2, CYP24A1, CYP26A1, CYP26B1, CYP26C1, CYP27A1, CYP27B1, CYP27C1, CYP39A1, CYP46A1 oder CYP51A1 umfasst.

8. Vorrichtung nach einem der Ansprüche 1-7, wobei das zweite Enzym aus der Gruppe bestehend aus einer Carbonyl-Reduktase, einer Aldehyd-Dehydrogenase, einer Arylalkohol-Dehydrogenase, einer Alkohol-Dehydrogenase, einer Pyruvat-Dehydrogenase, einer D-1-Xylose-Dehydrogenase, einer Oxoglutarat-Dehydrogenase, einer Isopropanol-Dehydrogenase, einer Glucose-6-phosphat-Dehydrogenase, einer Glucose-Dehydrogenase, einer Malat-Dehydrogenase, einer Formiat-Dehydrogenase, einer Benzaldehyd-Dehydrogenase, einer Glutamat-Dehydrogenase und einer Isocitrat-Dehydrogenase ausgewählt ist.

9. Vorrichtung nach einem der Ansprüche 1-8, wobei es sich bei dem Cofaktor um Nicotinamid-Adenin-Dinukleotid + Wasserstoff (NADH), Nicotinamid-Adenin-Dinukleotidphosphat + Wasserstoff (NADPH), Flavin-Adenin-Dinukleotid + Wasserstoff (FADH) oder Glutathion handelt.

10. Vorrichtung nach einem der Ansprüche 1-9, wobei das erste Enzym an Phase I beteiligt ist oder wobei die Vorrichtung ferner ein drittes Enzym umfasst, das am Phase-II-Stoffwechsel beteiligt ist.

11. Vorrichtung nach einem der Ansprüche 1-10, ferner umfassend ein viertes Enzym, das eine ROS (Reactive Oxygen Species) reduziert; gegebenenfalls wobei es sich bei dem vierten Enzym um eine Katalase, eine Superoxid-Dismutase (SOD) oder eine Glutathion-Peroxidase/Glutathiondisulfid-Reduktase oder eine Kombination davon handelt.

12. Vorrichtung nach einem der Ansprüche 1-11, ferner umfassend ein fünftes Enzym, das aus der Gruppe bestehend aus einer UDP-Glucuronosyltransferase, einer Sulfotransferase, einer Monoamin-Oxidase und einer Carboxylesterase ausgewählt ist.

13. Vorrichtung nach einem der Ansprüche 1-12, wobei es sich bei dem makroporösen Gerüst um ein magnetisches makroporöses Gerüst handelt;
a. gegebenenfalls wobei es sich bei dem makroporösen magnetischen Gerüst um ein polymeres Hybridgerüst handelt, das ein vernetztes wasserunlösliches Polymer und eine ungefähr gleichmäßige Verteilung eingebetteter magnetischer Mikropartikel (MMP) umfasst;
i. gegebenenfalls wobei das magnetische makroporöse polymere Hybridgerüst PVA und ein aus der Gruppe bestehend aus CMC, Alginat, HEC, EHEC ausgewähltes Polymer umfasst; oder
ii. gegebenenfalls wobei das magnetische makroporöse polymere Hybridgerüst ein hydrophiles Polymer umfasst; gegebenenfalls wobei es sich bei dem hydrophilen Polymer um Xanthan handelt.

14. Verfahren zur Erzeugung von Metaboliten einer Verbindung, umfassend Mischen der Verbindung mit einem diffusionsfähigen Substrat in einer Reaktionslösung, Positionieren der Vorrichtung nach einem der Ansprüche 1-13, so dass das die magnetischen Nanopartikel umfassende makroporöse Gerüst mit der Reaktionslösung in Kontakt kommt, und Messen eines Produkts, das aus einer enzymatischen Reaktion in der Reaktionslösung entsteht;
a. gegebenenfalls ferner umfassend den Schritt, bei dem das die Mikrosomen und die magnetischen Nanopartikel umfassende macroporöse Gerüst aus der Reaktionslösung entfernt wird;
b. gegebenenfalls wobei das Verfahren in ein Screening-Verfahren mit hohem Durchsatz für Screening der Toxizität mehrerer Verbindungen integriert ist; oder
c. gegebenenfalls wobei das Verfahren in ein Screening-Verfahren mit hohem Durchsatz für Screening von Metaboliten aus Gemischen von Stoffwechselenzymen integriert ist.

15. Verfahren zur Herstellung der Vorrichtung nach einem der Ansprüche 1-13, umfassend magnetisches Einfangen des zweiten Enzyms in den mesoporösen Aggregaten magnetischer Nnopartikel, Kombinieren der Aggregate mit den das erste Enzym umfassenden Mikrosomen, Schablonieren der Aggregate und Mikrosomen auf die makroporösen Gerüste und Schablonieren der Gerüste auf den nicht reaktiven Teil;
a. gegebenenfalls wobei die Aggregate ferner ein drittes Enzym mit einer Cofaktor-Reduktase-Aktivität umfassen;
i. gegebenenfalls wobei die Aggregate ferner ein viertes Enzym umfassen, bei dem es sich um eine Katalase, eine Superoxid-Dismutase (SOD) oder eine Glutathion-Peroxidase/Glutathiondisulfid-Reduktase handelt; oder
ii. gegebenenfalls wobei die Aggregate ferner ein fünftes Enzym umfassen, das am Phase-II-Stoffwechsel beteiligt ist.

## Revendications

1. Dispositif pour la conversion de substrats diffusibles dans des produits diffusibles, comprenant des microsomes et des agrégats mésoporeux auto-assemblés de nanoparticules magnétiques, une première enzyme requérant un cofacteur diffusible ayant une première activité enzymatique étant contenue à l'intérieur desdits microsomes, une deuxième enzyme comprenant une activité de régénération de cofacteur étant piégée de manière magnétique à l'intérieur desdits mésopores,
ledit cofacteur étant utilisé dans ladite première activité enzymatique ;
lesdites nanoparticules magnétiques étant associées à un échafaudage macroporeux ;
lesdits microsomes étant associés audit échafaudage macroporeux ; et
ledit échafaudage macroporeux comprenant lesdites nanoparticules magnétiques étant associé à une partie non réactive utilisable pour le placement dudit échafaudage macroporeux dans ou l'élimination de celui-ci d'une solution de réaction ;
a. éventuellement l'une ou plusieurs desdites enzymes étant produites par une technologie d'ADN recombinant ou étant synthétisées ; ou
b. éventuellement lesdites nanoparticules magnétiques comprenant des microsomes de foie humain (HLM), des enzymes provenant d'une fraction de cytosol de foie humain (HCLF), ou UGT1A6.

2. Dispositif selon la revendication 1, comprenant en outre une partie fonctionnelle pour le maintien de manière stable desdits microsomes, desdites nanoparticules magnétiques, de ladite première enzyme, de ladite deuxième enzyme et dudit échafaudage macroporeux ;
a. éventuellement ladite partie fonctionnelle comprenant un tampon ;
b. éventuellement ladite partie fonctionnelle comprenant un substrat pour ladite deuxième enzyme ;
c. éventuellement ladite partie fonctionnelle comprenant ledit cofacteur ;
d. éventuellement ladite partie fonctionnelle étant magnétique ;
e. éventuellement ledit cofacteur étant piégé dans lesdits agrégats mésoporeux de nanoparticules magnétiques avec lesdites première et deuxième enzymes ; ou
f. éventuellement, ledit échafaudage macroporeux étant sous la forme d'une broche cylindrique, d'un globe, d'une perle, d'une capsule, d'un cube, d'une tige carrée, d'une pyramide, d'un diamant, ou étant amorphe.

3. Dispositif selon l'une ou l'autre des revendications 1 et 2, ladite partie non réactive comprenant un métal, une matière plastique, une céramique, un composite, ou une combinaison correspondante ;
a. éventuellement ladite partie non réactive comprenant une poignée pour la manipulation dudit dispositif ;
b. éventuellement, ladite partie non réactive étant utilisable pour une manipulation par un bras robotique pour un criblage à haut débit ; ou
c. éventuellement ladite partie non réactive permettant la diffusion de gaz.

4. Dispositif selon l'une quelconque des revendications 1 à 3, lesdits agrégats mésoporeux de nanoparticules magnétiques ayant une composition d'oxyde de fer ;
a. éventuellement lesdits agrégats mésoporeux de nanoparticules magnétiques ayant une distribution de tailles de nanoparticules magnétiques dans laquelle au moins 90 % desdites nanoparticules magnétiques ont une taille d'au moins 3 nm et jusqu'à 30 nm, et une distribution de tailles de particules agrégées dans laquelle au moins 90 % desdits agrégats mésoporeux de nanoparticules magnétiques ont une taille d'au moins 10 nm et jusqu'à 500 nm ;
b. éventuellement lesdits agrégats mésoporeux de nanoparticules magnétiques possédant une magnétisation saturée d'au moins 10 emu/g ; ou
c. éventuellement, lesdits agrégats mésoporeux de nanoparticules magnétiques possédant une magnétisation résiduelle jusqu'à 5 emu/g.

5. Dispositif selon l'une quelconque des revendications 1 à 4, lesdites première et deuxième enzymes étant contenues dans lesdits agrégats mésoporeux de nanoparticules magnétiques en une quantité jusqu'à 100 % de capacité de saturation ; éventuellement lesdites première et deuxième enzymes étant physiquement inaccessibles à des microbes.

6. Dispositif selon l'une quelconque des revendications 1 à 5, ladite première enzyme étant une enzyme oxydante ; éventuellement ladite enzyme oxydante étant une oxygénase contenant une flavine ; ladite composition comprenant en outre une troisième enzyme ayant une activité de cofacteur réductase qui est co-localisée avec ladite première enzyme ; éventuellement ladite troisième enzyme étant une cytochrome P450 réductase.

7. Dispositif selon la revendication 6, ladite enzyme oxydante étant une P450 monooxygénase ;
a. éventuellement une unique protéine comprenant ladite P450 monooxygénase et ladite troisième enzyme ;
b. éventuellement, ladite P450 monooxygénase étant co-localisée avec ladite troisième enzyme à l'intérieur d'une membrane lipidique ;
c. éventuellement ladite P450 monooxygénase comprenant une séquence de P450 qui est mammifère ; éventuellement ladite P450 monooxygénase comprenant une séquence de P450 qui est humaine ;
d. éventuellement ladite P450 monooxygénase comprenant CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1, CYP3A4, CYP3A5, CYP3A7, CYP3A43, CYP4A11, CYP4A22, CYP4B1, CYP4F2, CYP4F3, CYP4F8, CYP4F11, CYP4F12, CYP4F22, CYP4V2, CYP4X1, CYP4Z1, CYP5A1, CYP7A1, CYP7B1, CYP8A1, CYP8B1, CYP11A1, CYP11B1, CYP11B2, CYP17A1, CYP19A1, CYP20A1, CYP21A2, CYP24A1, CYP26A1, CYP26B1, CYP26C1, CYP27A1, CYP27B1, CYP27C1, CYP39A1, CYP46A1, ou CYP51A1.

8. Dispositif selon l'une quelconque des revendications 1 à 7, ladite deuxième enzyme étant choisie dans le groupe constitué par une carbonyle réductase, une aldéhyde déshydrogénase, une arylalcool déshydrogénase, une alcool déshydrogénase, une pyruvate déshydrogénase, une D-1 xylose déshydrogénase, une oxoglutarate déshydrogénase, une isopropanol déshydrogénase, une glucose-6-phosphate déshydrogénase, une glucose déshydrogénase, une malate déshydrogénase, une formiate déshydrogénase, une benzaldéhyde déshydrogénase, une glutamate déshydrogénase et une isocitrate déshydrogénase.

9. Dispositif selon l'une quelconque des revendications 1 à 8, ledit cofacteur étant le nicotinamide adénine dinucléotide + hydrogène (NADH), le nicotinamide adénine dinucléotide phosphate + hydrogène (NADPH), la flavine adénine dinucléotide + hydrogène (FADH) ou le glutathion.

10. Dispositif selon l'une quelconque des revendications 1 à 9, ladite première enzyme participant à la phase I ou le dispositif comprenant en outre une troisième enzyme qui participe au métabolisme de phase II.

11. Dispositif selon l'une quelconque des revendications 1 à 10, comprenant en outre une quatrième enzyme qui réduit une espèce d'oxygène réactive (ROS) ; éventuellement ladite quatrième enzyme étant une catalase, une superoxyde dismutase (SOD) ou une glutathion peroxydase/glutathion-disulfure réductase ou une combinaison correspondante.

12. Dispositif selon l'une quelconque des revendications 1 à 11, comprenant en outre une cinquième enzyme choisie dans le groupe constitué par une UDP-glucoronosyl transférase, une sulfotransférase, une monoamine oxydase et une carboxylestérase.

13. Dispositif selon l'une quelconque des revendications 1 à 12, ledit échafaudage macroporeux étant un échafaudage macroporeux magnétique ;
a. éventuellement ledit échafaudage magnétique macroporeux étant un échafaudage hybride polymérique comprenant un polymère insoluble dans l'eau réticulé et une distribution approximativement uniforme de microparticules magnétiques incorporées (MMP) ;
i. éventuellement ledit échafaudage hybride polymérique macroporeux magnétique comprenant un PVA est un polymère choisi dans le groupe constitué par CMC, alginate, HEC, EHEC ; ou
ii. éventuellement ledit échafaudage hybride polymérique macroporeux magnétique comprenant un polymère hydrophile ; éventuellement ledit polymère hydrophile étant une gomme xanthane.

14. Procédé de production de métabolites d'un composé, comprenant le mélange dudit composé avec un substrat diffusible dans une solution de réaction, le positionnement du dispositif selon l'une quelconque des revendications 1 à 13 de sorte que ledit échafaudage macroporeux comprenant lesdites nanoparticules magnétiques entre en contact avec ladite solution de réaction, et la mesure d'un produit résultant d'une réaction enzymatique dans ladite solution de réaction ;
a. éventuellement comprenant en outre l'étape d'élimination dudit échafaudage macroporeux comprenant lesdits microsomes et lesdites nanoparticules magnétiques depuis ladite solution de réaction ;
b. éventuellement, ledit procédé étant incorporé dans un procédé de criblage à haut débit pour le criblage de la toxicité d'une pluralité de composés ; ou
c. éventuellement ledit procédé étant incorporé dans un procédé de criblage haut débit pour le criblage de métabolites à partir de mélanges d'enzymes métaboliques.

15. Procédé de fabrication du dispositif selon l'une quelconque des revendications 1 à 13, comprenant le piégeage de manière magnétique de ladite deuxième enzyme dans lesdits agrégats mésoporeux de nanoparticules magnétiques, comprenant lesdits agrégats comprenant ladite deuxième enzyme avec lesdits microsomes comprenant ladite première enzyme, le matriçage desdits agrégats et microsomes sur lesdits échafaudages macroporeux, et le matriçage desdits échafaudages sur ladite partie non réactive ;
a. éventuellement lesdits agrégats comprenant en outre une troisième enzyme ayant une activité de cofacteur réductase ;
i. éventuellement lesdits agrégats comprenant en outre une quatrième enzyme qui est une catalase, une superoxyde dismutase (SOD) ou une glutathion peroxydase/glutathion-disulfure réductase ; ou
ii. éventuellement lesdits agrégats comprenant en outre une cinquième enzyme qui participe au métabolisme de phase II.
